(19) **Europäisches Patentamt**
**European Patent Office**
**Office européen des brevets**

(11) **EP 2 649 945 B1**

(12) **EUROPEAN PATENT SPECIFICATION**

(45) Date of publication and mention
of the grant of the patent:
**23.11.2016 Bulletin 2016/47**

(51) Int Cl.:
***A61B 10/00*** *(2006.01)*    ***A61B 5/16*** *(2006.01)*
***A61B 5/00*** *(2006.01)*

(21) Application number: **11847764.5**

(22) Date of filing: **01.12.2011**

(86) International application number:
**PCT/JP2011/006754**

(87) International publication number:
**WO 2012/077313 (14.06.2012 Gazette 2012/24)**

(54) **DEVICE FOR VERIFYING ONSET OF DEMENTIA**

VORRICHTUNG ZUR ÜBERPRÜFUNG DES BEGINNS VON DEMENZ

DISPOSITIF DE VÉRIFICATION DE L'APPARITION DE LA DÉMENCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.12.2010 JP 2010271373**

(43) Date of publication of application:
**16.10.2013 Bulletin 2013/42**

(73) Proprietor: **National University Corporation
Okayama University
Kita-ku
Okayama-shi
Okayama 700-8530 (JP)**

(72) Inventors:
 • **WU, Jinglong
  Okayama-shi
  Okayama 700-8530 (JP)**
 • **ABE, Koji
  Okayama-shi
  Okayama 700-8558 (JP)**

 • **LI, Chunlin
  Okayama-shi
  Okayama 700-8530 (JP)**

(74) Representative: **Müller-Boré & Partner
Patentanwälte PartG mbB
Friedenheimer Brücke 21
80639 München (DE)**

(56) References cited:
 JP-A- 2002 112 981    JP-A- 2003 079 631
 JP-A- 2004 135 824    JP-A- 2004 527 295
 JP-A- 2005 192 637    JP-A- 2006 158 421
 JP-A- 2006 158 944    JP-A- 2010 158 523
 US-A1- 2002 143 240

 • JENNIFER L MOZOLIC ET AL: "Modality-specific
 selective attention attenuates multisensory
 integration", EXPERIMENTAL BRAIN
 RESEARCH, SPRINGER, BERLIN, DE, vol. 184,
 no. 1, 8 August 2007 (2007-08-08), pages 39-52,
 XP019559979, ISSN: 1432-1106, DOI:
 10.1007/S00221-007-1080-3

## Description

Technical Field

The present invention relates to a method and a device for verifying onset of dementia.

Background Art

Heretofore, a method described in Patent Document 1 is known as a method for measuring cognitive ability of a brain, for example.

The cognitive ability measurement method described in Patent Document 1 is a method described below. First, a deteriorated image in which information for perceiving a meaningful object (e.g., a horse) is deteriorated by performing predetermined processing for an original image having the object is prepared, and an image display unit displays this deteriorated image for a predetermined time. Next, a perception time in which the subject looking at the deteriorated image perceives the object (perceive that the object is a horse) is measured. Then, a position of the subject among a subject group is shown on the basis of the measured perception time, and perception times previously measured for a large number of subjects.

However, in the method of Patent Document 1, there is a possibility that since a time for perceiving the object is measured, the perception time cannot be accurately measured depending on the degree of interest or concern of the subject to the object.

Specifically, in a case of measuring a perception time for an object that the subject has little interest or concern, it cannot be identified whether the perception time is long on the basis of decline of the cognitive ability of the brain, or the perception time is long due to originally visual difficulty for the subject.

Patent Document 1: Japanese Unexamined Patent Pablication No. 2006-87743

J L MOZOLIC ET AL: "Modality-specific selective attention attenuates multisensory integration", EXPERIMENTAL BRAIN RESEARCH, SPRINGER, BERLIN, DE, vol. 184, no. 1, 8 August 2007, pages 39-52 discloses a device for detecting dementia by comparing how selective attention to a single sensory modality (auditory or visual) and divided attention (between auditory and visual modalities) modulate the integration of semantically matching multisensory stimuli.

Summary of the Invention

An object of the present invention has been conceived in order to solve the aforementioned problems, and is to provide a method and a device capable of obtaining information for verifying onset of dementia while suppressing individual differences between subjects.

In order to solve the problems, the present invention provides a method for verifying onset of dementia of a subject, this method including: a stimulus provision step of providing the subject with one of two alternatively selectable selection items as a stimulus including at least one of audio information and visual information, a plurality of times; a measurement step of receiving a response from the subject with respect to the stimulus provided in the stimulus provision step, and measuring a response time from the provision of the stimulus to the reception of the response; an average value computation step of computing an average value of the response time measured in the measurement step; a preparation step of previously preparing reference average values with respect to a plurality of subjects including a dementia patient and a person other than the dementia patient, the reference average values being average values of the response times to the stimulus provided in the stimulus provision step; and a comparison step of comparing the average value of the response times obtained with respect to a specified subject in the average value computation step with the reference average values.

Additionally, the present invention provides a device for verifying onset of dementia for a subject, which includes: an information provision unit capable of providing the subject with one of two alternatively selectable selection items as a stimulus including at least one of audio information and visual information; an operation unit capable of outputting, in accordance with operation from the subject, a command showing that one of the two selection items is selected; and a control unit controlling the information provision unit in order to provide the subject with the selection item, and analyzes information for verifying the onset of dementia on the basis of the command input from the operation unit, wherein the control unit has: a stimulus provision unit which provides a stimulus showing one of the two selection items through the information provision unit a plurality of times; a measurement unit which measures a response time from the provision of the stimulus by the stimulus provision unit to the reception of the command output from the operation unit; an average time computation unit which computes an average value of the response times measured by the measurement unit; a storage unit which previously stores reference average values previously prepared with respect to a plurality of subjects including a dementia patient and a person other than the dementia patient, the reference average values being average values of the response times to the stimulus provided by the stimulus provision unit; and a data creation unit which creates data, in which the average value of the response times obtained with respect to a specified subject by the average time computation unit is compared with the reference average values.

The present invention can obtain the information for verifying the onset of dementia while suppressing individual differences between subjects.

Brief Description of the Drawings

[Fig. 1] Fig. 1 is a schematic diagram showing a whole configuration of a device for verifying onset of dementia according to a first embodiment of the present invention.

[Fig. 2] Fig. 2 is a block diagram showing an electrical configuration of a control unit of Fig. 1.

[Fig. 3] Figs. 3 are a set of image diagrams displayed by the control unig of Fig. 2, in which Fig. 3A shows an audio selective attention condition, Fig. 3B shows a visual selective attention condition, and Fig. 3C shows an audio-visual division attention condition.

[Fig. 4] Fig. 4 is a schematic diagram showing a state where a visual stimulus is provided by the control unit of Fig. 2.

[Fig. 5] Fig. 5 is a schematic diagram showing a state where an audio stimulus is provided by the control unit of Fig. 2.

[Fig. 6] Fig. 6 is a schematic diagram showing a state where an audio-visual stimulus is provided by the control unit of Fig. 2.

[Fig. 7] Fig. 7 is a timing chart showing stimulus provision timing and stimulus stop timing.

[Fig. 8] Fig. 8 is a graph showing average values (reference average values) of response times by group, stored in a storage unit of Fig. 2.

[Fig. 9] Fig. 9 is a table showing existence or non-existence of significant differences between respective groups in the average values of the response times stored in the storage unit of Fig. 2.

[Fig. 10] Fig. 10 is a graph showing percentages of correct answers (reference percentage of correct answers) by group, stored in the storage unit of Fig. 2.

[Fig. 11] Fig. 11 is a table showing existence or non-existence of significant differences between respective groups in the percentages of correct answers stored in the storage unit of Fig. 2.

[Fig. 12] Fig. 12 is a graph showing cumulative distribution probabilities obtained on the basis of the response times in the audio selective attention condition and the visual selective attention condition.

[Fig. 13] Fig. 13 is a schematic diagram for illustrating a race model.

[Fig. 14] Fig. 14 is a graph showing cumulative distribution probabilities obtained on the basis of the response times in the audio-visual division attention condition.

[Fig. 15] Fig. 15 is a graph showing promoting effect indexes under the respective conditions, obtained by using the cumulative distribution probabilities of Fig. 13 and Fig. 14.

[Fig. 16] Fig. 16 is a graph showing promoting effect indexes under the audio selective attention condition for the respective groups, stored in the storage unit of Fig. 2.

[Fig. 17] Fig. 17 is a graph showing promoting effect indexes under the visual selective attention condition for the respective groups, stored in the storage unit of Fig. 2.

[Fig. 18] Fig. 18 is a graph showing promoting effect indexes under the audio-visual division attention condition for the respective groups, stored in the storage unit of Fig. 2.

[Fig. 19] Fig. 19 is a flowchart showing processing performed by the control unit of Fig. 2.

[Fig. 20] Fig. 20 is a flowchart showing information collecting processing of Fig. 19.

[Fig. 21] Fig. 21 is a flowchart showing processes of an audio selective attention condition test of Fig. 20.

[Fig. 22] Fig. 22 is a flowchart showing processes of a visual selective attention condition test of Fig. 20.

[Fig. 23] Fig. 23 is a flowchart showing processes of an audio-visual division attention condition test of Fig. 20.

[Fig. 24] Fig. 24 is a flowchart showing stimulus provision processing shown in Fig. 21 to Fig. 23.

[Fig. 25] Fig. 25 is a flowchart showing information analysis processing shown in Fig. 19.

[Fig. 26] Fig. 26 is a schematic diagram showing a whole configuration of a device for verifying onset of dementia according to a second embodiment of the present invention.

[Fig. 27] Fig. 27 is a block diagram showing an electrical configuration of a control unit of Fig. 25.

[Fig. 28] Fig. 28 is an image diagram displayed by the control unit of Fig. 27.

[Fig. 29] Fig. 29 is a timing chart of a task performed by the control unit of Fig. 27.

[Fig. 30] Fig. 30 is a schematic diagram showing the ratio of validity cue stimuli to invalidity cue stimuli included in cue stimuli in Fig. 29.

[Fig. 31] Fig. 31 is a graph showing reference average values stored by the control unit of Fig. 27.

[Fig. 32] Fig. 32 is a table showing information of a subject for which the response times based on the reference average values of Fig. 31 are measured.

[Fig. 33] Fig. 33 is a flowchart showing information collecting processing T performed by the control unit of Fig. 27.

[Fig. 34] Fig. 34 is a flowchart showing response reception processing Z of Fig. 33.

[Fig. 35] Fig. 35 is a flowchart showing information analysis processing U.

[Fig. 36] Fig. 36 is a schematic diagram showing a modification of the verification device of Fig. 26.

[Fig. 37] Fig. 37 is a block diagram showing an electrical configuration of the control unit of Fig. 36.

[Fig. 38] Fig. 38 shows activation sites of a brain and BOLD signal change rates analyzed by the control unit of Fig. 36, which are measurement results (1/2) at an interval of 200 ms.

[Fig. 39] Fig. 39 shows the activation sites of the brain and BOLD signal change rates analyzed by the control unit of Fig. 36, which are measurement results (2/2) at an interval of 200 ms.

[Fig. 40] Fig. 40 shows the activation sites of the brain and BOLD signal change rates analyzed by the control unit of Fig. 36, which are measurement results at an interval of 400 ms.

[Fig. 41] Fig. 41 shows the activation sites of the brain and BOLD signal change rates analyzed by the control unit of Fig. 36, which are measurement results (1/2) at an interval of 800 ms.

[Fig. 42] Fig. 42 shows the activation sites of the brain and BOLD signal change rates analyzed by the control unit of Fig. 36, which are measurement results (2/2) at an interval of 800 ms.

[Fig. 43] Fig. 43 is a flowchart showing a part of information collecting processing T performed by the control unit of Fig. 36.

[Fig. 44] Fig. 44 is a flowchart showing information analysis processing U performed by the control unit of Fig. 36.

Modes for Carrying Out the Invention

With reference to the attached drawings, embodiments of the present invention will be hereinafter described. The following embodiments are each an embodied example of the present invention, and do not restrict the technical scope of the present invention.

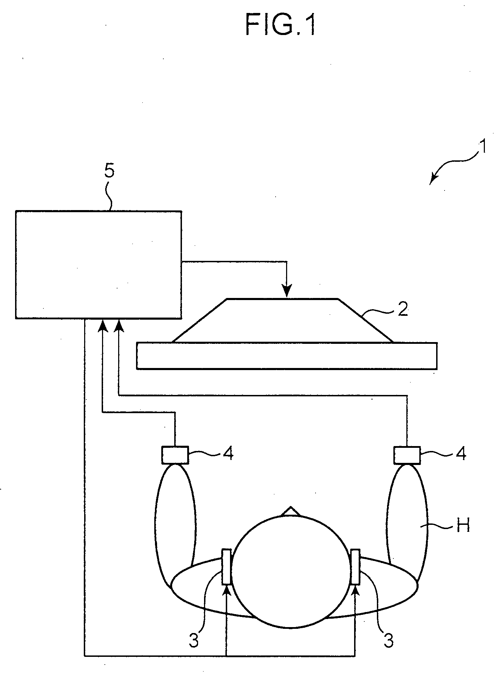

Fig. 1 is a schematic diagram showing a whole configuration of a device for verifying onset of dementia according to a preferred embodiment of the present invention.

With reference to Fig. 1, a verification device 1 includes a display (image display device: visual information provision unit) 2 capable of providing a subject H with visual information, a pair of right and left ear pad units (audio information provision units) 3 of headphones capable of outputting audio information to the subject H, a pair of right and left operation units 4 which outputs a command in accordance with operation from the subject H, a control unit 5 electrically connected to the display 2, the ear pad units 3, and the operation units 4.

The display 2 displays a preset image in accordance with a command from the control unit 5.

The ear pad units 3 each output preset sound in accordance with the command from the control unit 5. Specifically, the ear pad units 3 are configured so as not only to be able to output sound from both the ear pad units 3 simultaneously, but also to be able to output sound from only one of the ear pad units 3.

The operation units 4 are configured from respective push buttons. Specifically, the operation units 4 can each output, to the control unit 5 in accordance with push operation from the subject, a command indicating that the push operation is received.

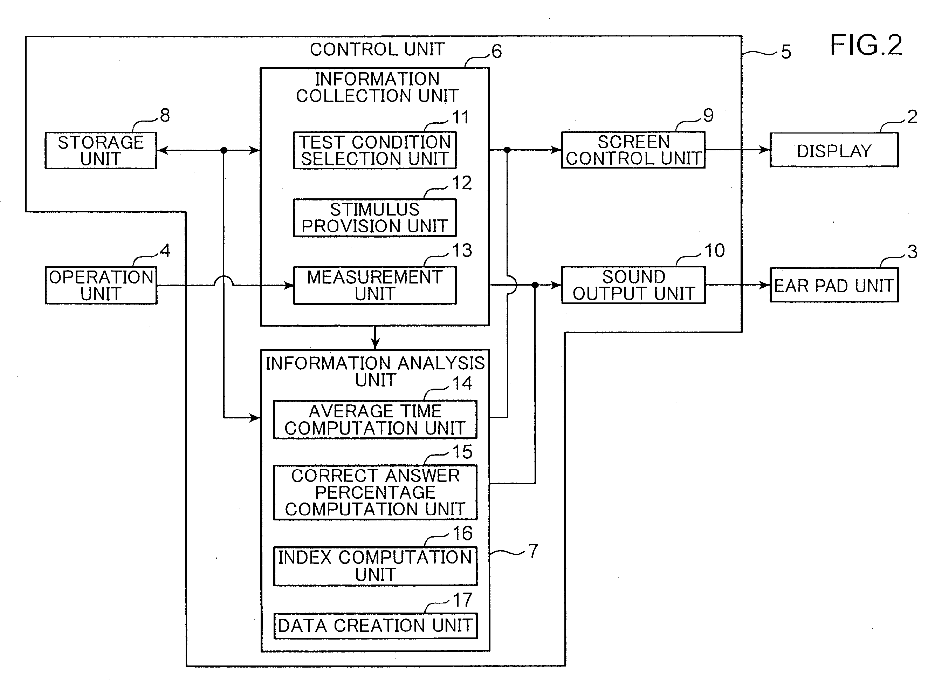

Fig. 2 is a block diagram showing an electrical configuration of the control unit of Fig. *1.

The control unit 5 controls the image display by the display 2, and the sound output by the ear pad units 3, and analyzes preset information on the basis of commands from the operation units 4.

Specifically, the control unit 5 includes an information collection unit 6 for collecting information for verifying the onset of dementia, an information analysis unit 7 which analyzes the information collected by the information collection unit 6, a storage unit 8 which stores information for being compared with the information analyzed by the information analysis unit 7, a screen control unit 9 which controls the image display by the display 2 in accordance with commands from the information collection unit 6 and the information analysis unit 7, and a sound output unit 10 which controls, in accordance with commands from the information collection unit 6 and the information analysis unit 7, the sound output by the command ear pad units 3.

The information collection unit 6 provides at least one of visual information and audio information through the display 2 and the ear pad units 3, and receives a response (command from the operation units 4) from the subject H in response to this information provision. Specifically, the information collection unit 6 includes a test condition selection unit 11 which selects a test condition, a stimulus provision unit 12 which provides a stimulus under a condition selected by the test condition selection unit 11, and a measurement unit 13 which measures a response time to the stimulus provided by the stimulus provision unit 12.

The test condition selection unit 11 selects, as a test condition, a condition from three kinds of conditions, namely an audio selective attention condition, a visual selective attention condition, and an audio-visual division attention condition. The audio selective attention condition is a condition in which attention is paid only to information obtained through an auditory sense to respond. The visual selective attention condition is a condition in which attention is paid only to information obtained through a visual sense to respond. The audio-visual division attention condition is a condition in which attention is paid only to information obtained through the auditory sense and the visual sense to respond.

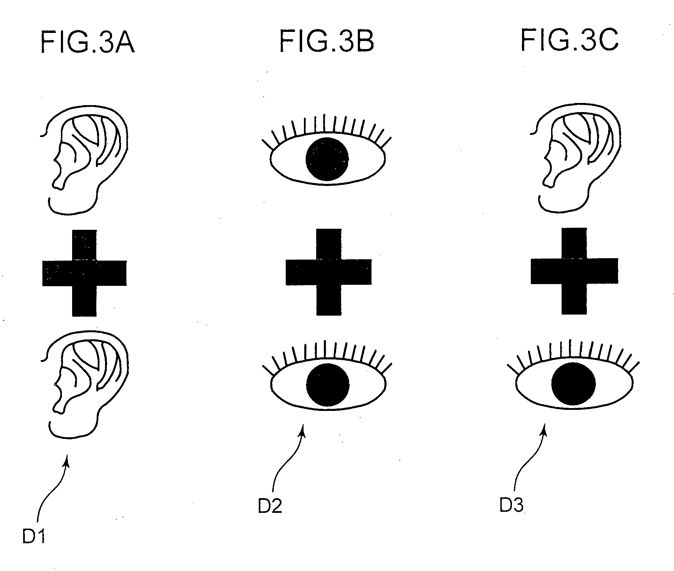

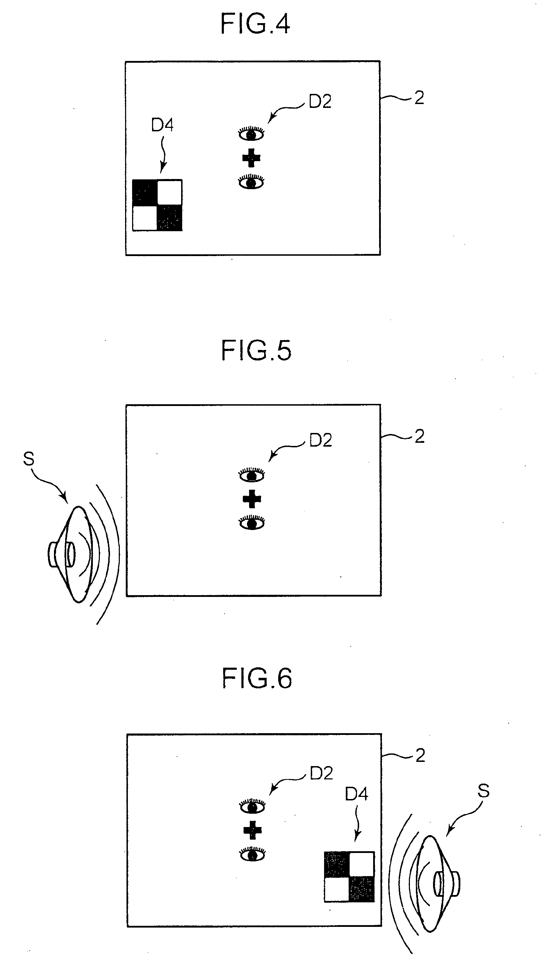

The test condition selection unit 11 displays a condition image D1 shown in Fig. 3A on the display 2, in a case where the audio selective attention condition is selected as the test condition. The condition image D1 has images showing two ears arranged vertically, and an image of "+" arranged between these images. The test condition selection unit 11 displays a condition image D2 shown in Fig. 3B on the display 2, in a case where the visual selective attention condition is selected as the test condition. The condition image D2 has images showing two eyes arranged vertically, and an image of "+" arranged between these images. The test condition selection unit 11 displays a condition image D3 shown in Fig. 3C on the display 2, in a case where the audio-visual division attention condition is selected as the test condition. The condition image D3 has an image showing an ear, and image showing an eye arranged below this image, and an image of "+" arranged between these images. These condition images D 1 to D3 are displayed on a center of the display 2, as shown in Fig. 4 to Fig. 6.

The stimulus provision unit 12 offers the test condition selected by the test condition selection unit 11 to the subject H, and provides the subject H with three kinds of stimuli, namely an audio stimulus, a visual stimulus, and an audio-visual stimulus. The audio stimulus is information provided through the ear pad units 3. The visual stimulus is information provided through the display 2. The audio-visual stimulus is information provided through the ear pad units 3 and the display 2. Hereinafter, the audio stimulus, the visual stimulus and the audio-visual stimulus provided by the stimulus provision unit 12 will be described with reference to Fig. 4 to Fig. 6.

The stimulus provision unit 12 outputs one of two alternatively selectable selection items from the ear pad units 3 as the audio stimulus. Specifically, the stimulus provision unit 12 outputs the sound S only from the left ear pad unit 3, as shown in Fig. 5, or outputs sound S only from the right ear pad unit 3, as shown in Fig. 6. That is, in this embodiment, the 'two alternatively selectable selection items' are items capable of being selected either left or right. The sound S has a frequency of 1000 Hz, and sound pressure of 70 dB.

The stimulus provision unit 12 displays one of two alternatively selectable selection items as the visual stimulus on the display 2. Specifically, the stimulus provision unit 12 displays a stimulus image D4 on a right or left side of the condition images D 1 to D3, as shown in Fig. 4 or Fig. 6. The stimulus image D4 is an image formed by blacking out two squares located diagonally among four squares formed by dividing a square with segments connecting midpoints of two pairs of facing sides of the square.

The stimulus provision unit 12 outputs one of the two alternatively selectable selection items from the ear pad units 3, and displays one of the two alternatively selectable selections on the display 2, as the audio-visual stimulus. Specifically, in a case of providing a right stimulus, the stimulus provision unit 12 outputs the sound S only from the right ear pad unit 3, and displays the stimulus image D4 on the right side of the condition images D1 to D3 on the display 2, as shown in Fig. 6. On the other hand, in a case of providing a left stimulus, the stimulus provision unit 12 outputs the sound S only from the left ear pad unit 3, and displays the stimulus image D4 on the left side of the condition images D 1 to D3 on the display 2. In the audio-visual stimulus, display of the stimulus image D4 and output of the sound S are continuously performed in the same period.

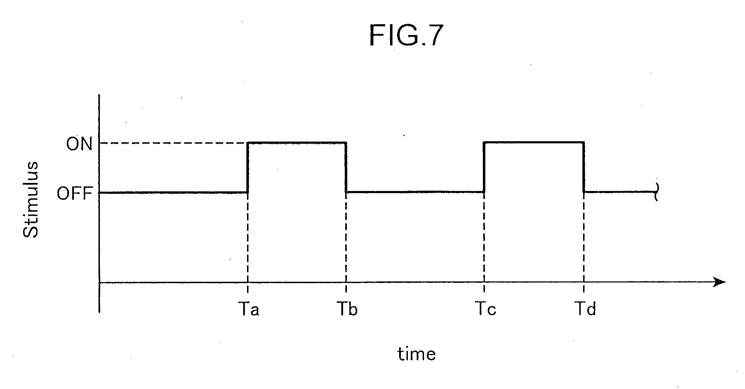

The stimulus provision unit 12 provides the three kinds of stimuli at an interval of a preset response period a plurality of times. Specifically, the stimulus provision unit 12 stops providing the stimulus from test start to a time point Ta (e.g., for five seconds), as shown in Fig. 7. Next, the stimulus provision unit 12 provides the stimulus during a preset period (e.g., 150 ms) from the time point Ta, and stops providing the stimulus at a time point Tb. A period between the time point Ta and the time point Tc is the response period (e.g., for 3 to 4 seconds) of the subject H. Then, the stimulus provision unit 12 provides the stimulus during a period between a time point Tc and a time point Td, similarly to the period between the time point Ta and the time point Tb. Thereafter, the stimulus provision period and the stimulus stop period are repeated.

Referring to Fig. 2 again, the measurement unit 13 measures the response time from the start of stimulus provision to the response of the subject H. Specifically, in a case of receiving the command from the operation units 4 from the provision start time point of the stimulus (e.g., time point Ta of Fig. 7) to the end time point of the response period (e.g., time point Tb of Fig. 7), the measurement unit 13 measures the response time from the stimulus provision start time point to command reception time point.

The information analysis unit 7 computes information necessary for verifying the onset of dementia on the basis of the response time obtained by the information collection unit 6, and compares the computed information with information stored in the storage unit 8.

Specifically, the information analysis unit 7 includes an average time computation unit 14 which computes an average value of the response times, a correct answer percentage computation unit 15 which computes a percentage of correct answers in the responses from the subject H, an index computation unit 16 which computes an index of a promoting effect by audio-visual integration, and a data creation unit 17 which creates data in which information computed by the respective computation units 14 and 15 is compared with the information stored in the storage unit 8.

The average time computation unit 14 computes average values of the response times for the respective three kinds of stimuli in each of the three kinds of conditions. Responses obtained by incorrect selection and responses obtained by operation of the both operation units 4 in the response periods among responses from the subject H are excluded from responses for computing the average values. Additionally, the average time computation unit 14 obtains a standard deviation SD and an average response time MRT of measurement values of a plurality of response times to the same stimulus in the same condition, and excludes a response time RT falling under the following formula (1) and formula (2) from the response times for computing the average values.

$$\mathrm{MRT} + 2 \times \mathrm{SD} < \mathrm{RT} \ \dots (1)$$

$$\mathrm{MRT} - 2 \times \mathrm{SD} > \mathrm{RT} \ \dots (2)$$

It is correct selection not to respond to the visual stimulus in the audio selective attention condition, and the audio stimulus in the visual selective attention condition. Therefore, the average values of the response times to the visual stimulus in the audio selective attention condition, and the audio stimulus in the visual selective attention condition are not computed.

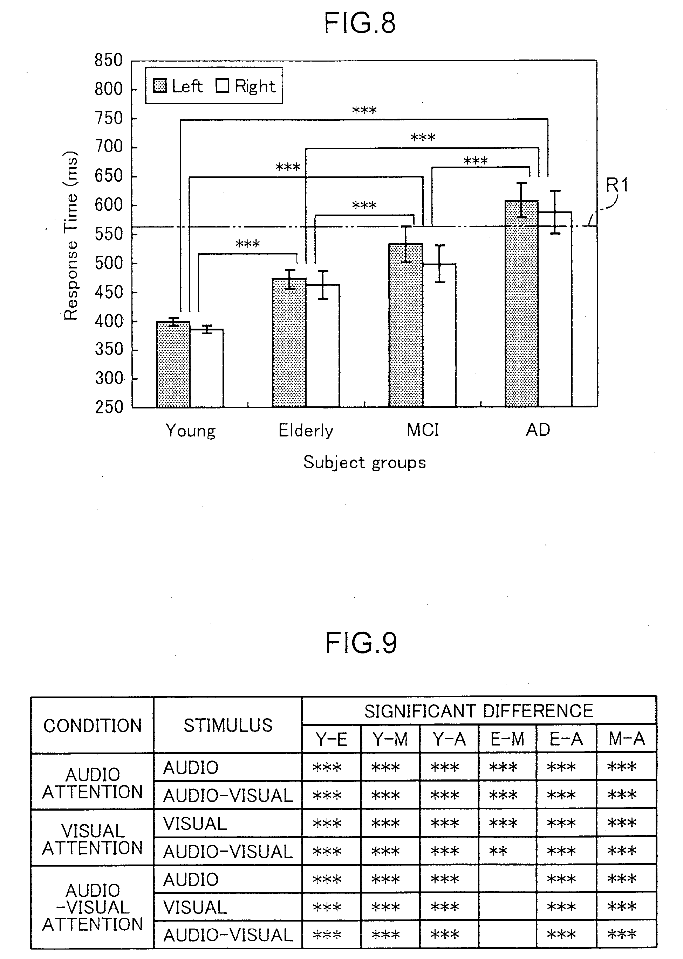

Here, the storage unit 8 stores information shown in Fig. 8 and Fig. 9 for comparison with the average values computed by the average time computation unit 14. Specifically, the storage unit 8 stores reference average values being average values of response times previously computed for a plurality of subjects H belonging to four groups including dementia patients (denoted by AD), people with mild cognitive impairment (denoted by MCI), healthy elderly people (denoted by Elderly), healthy young people (denoted by Young), as shown in Fig. 8. The respective reference average values have average values of the response times for the respective stimuli in the three kinds of conditions. Fig. 8 illustrates reference average values to the audio stimulus in the audio selective attention condition. In the reference average values shown in Fig. 8, the respective average response times of Young, Elderly, MCI, AD are longer in this order. As shown by "*" of Fig. 8 and Fig. 9, significant differences in the reference average value exist between the respective groups. "***" denotes a case where the significant difference is shown at P value < 0.001 in the result of a two way factorial analysis of variance. "**" denotes a case where the significant difference is shown at P value < 0.01 in the result of two way factorial analysis of variance. "*" denotes a case where the significant difference is shown at P value < 0.05 in the result of a two way factorial analysis of variance. In Fig. 9, "Y" means Young, "E" means Elderly, and "M" means MCI, and "A" means AD. As shown in Fig. 9, the significant differences at P value < 0.001 exists between the response times of the AD patients and the response times of people other than the AD patients. A test for obtaining the reference average values of this embodiment is performed with respect to subjects including AD patients of an average age of 73.6 between the ages of 57 and 83, MCI patients of an average age of 69.7 between the ages of 55 and 78, Elderly of an average age of 71.5 between the ages of 67 and 76, and Young of an average age of 22.5 between the ages of 21 and 25.

The data creation unit 17 (see Fig. 2) creates data in which an average value of the response times computed for the specified subject H is compared with the average value to the same stimulus in the same condition among the reference average values (see Fig. 8) stored in the storage unit 8. Specifically, the data creation unit 17 creates a graph, in which an average value R1 of the response times computed for the specified subject H is shown on a graph of the reference average values, as shown in Fig. 8, and displays this graph on the display 2.

The correct answer percentage computation unit 15 (see Fig. 2) computes percentages of correct answers for the respective three kinds of stimuli in each of the three kinds of conditions. The responses obtained by operation of the both operation units 4 in the response periods among the responses from the subjects H are treated as incorrect responses. It is correct selection not to respond to the visual stimulus in the audio selective attention condition, and the audio stimulus in the visual selective attention condition (no positive indication of intention). Therefore, the percentages of correct answers to the visual stimulus in the audio selective attention condition and the audio stimulus in the visual selective attention condition are not computed.

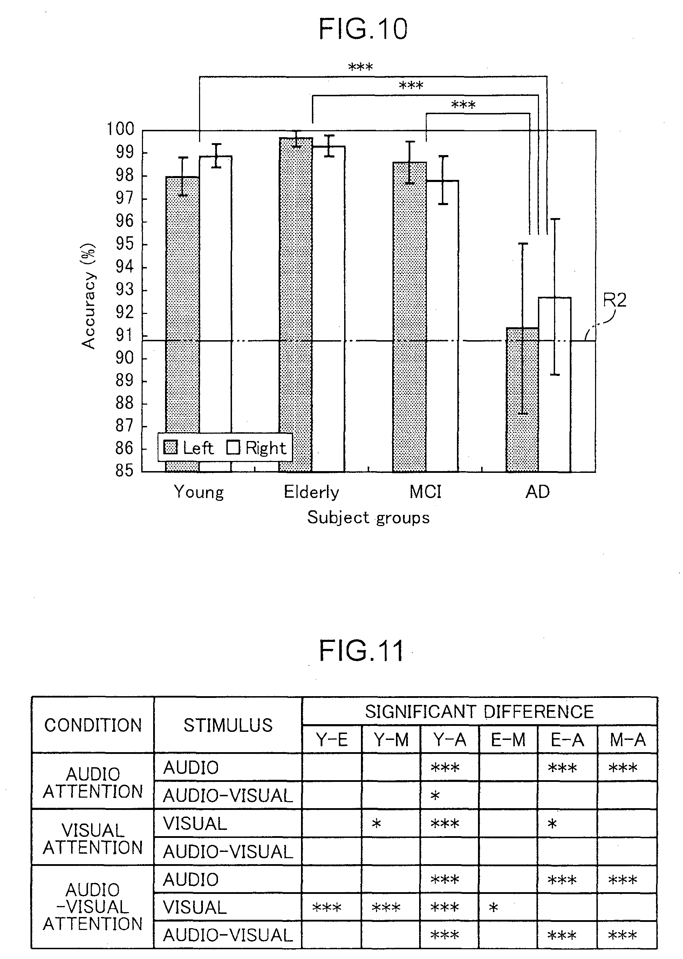

Here, the storage unit 8 stores information shown in Fig. 10 and Fig. 11 for comparison with the average values computed by the correct answer percentage computation unit 15. Specifically, the storage unit 8 stores reference percentages of correct answers being percentages of correct answers previously computed for a plurality of subjects H belonging to four groups including dementia patients (denoted by AD), people with mild cognitive impairment (denoted by MCI), healthy elderly people (denoted by Elderly), healthy young people (denoted by Young), as shown in Fig. 10. The respective reference average values have percentages of correct answers in the response times to the respective stimuli in the three kinds of conditions. Fig. 10 illustrates reference percentages of correct answers to the audio stimulus in the audio selective attention condition. As shown by "*" mark of Fig. 10 and Fig. 11, significant differences in the reference percentage of correct answers exist between the respective groups. Particularly, as shown in Fig. 11, in the reference percentages of correct answers, a plurality of the significant differences exist between the percentage of correct answers of the AD patients and the percentage of correct answers of the people other than the AD patients among combinations of the respective stimuli in the three kinds of conditions.

The data creation unit 17 (see Fig. 2) creates data in which a percentage of correct answers computed for the specified subject H is compared with the percentage of correct answers to the same stimulus in the same condition among the reference percentages of correct answers stored in the storage unit 8 (see Fig. 10). Specifically, the data creation unit 17 creates a graph in which a correct answer percentage R2 computed for the specified subject H is shown on a graph of the reference percentages of correct answers as shown in Fig. 10, and displays this graph on the display 2.

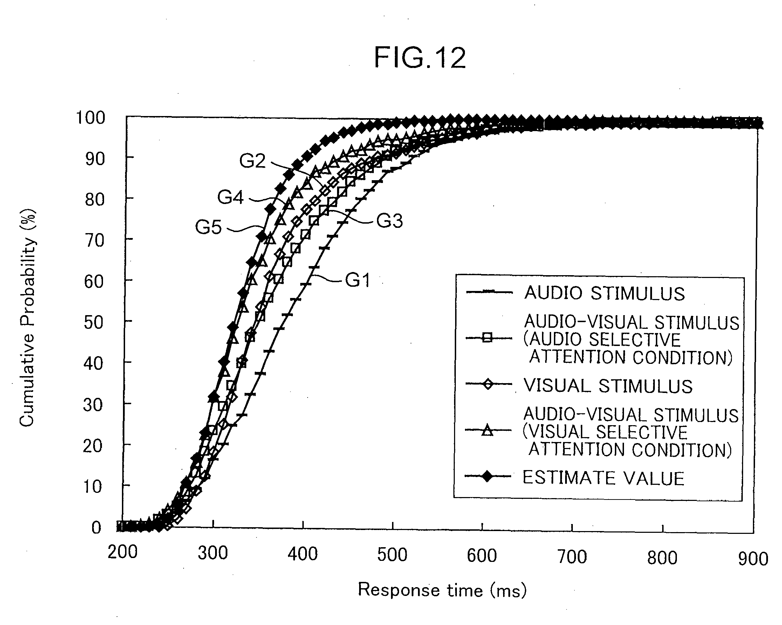

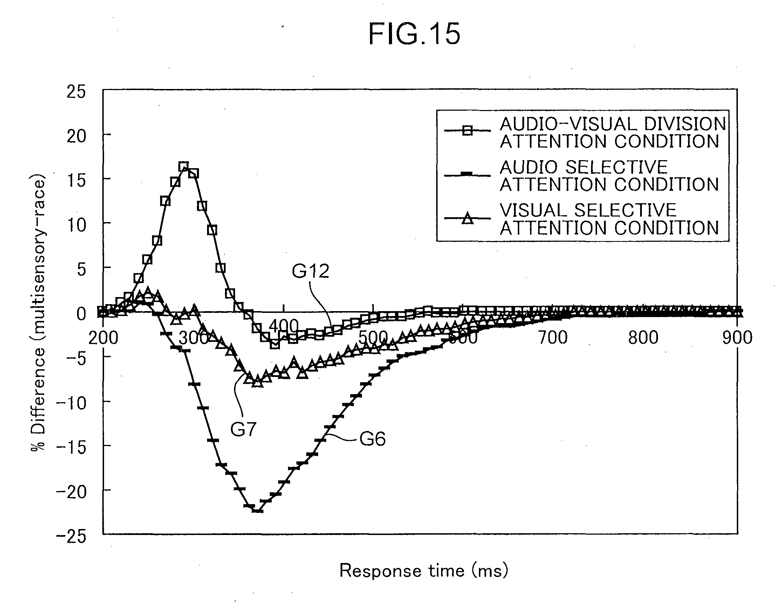

Referring to Fig. 2 again, the index computation unit 16 computes an index of a promoting effect by audio-visual integration for the specified subject H. Specifically, the index computation unit 16 computes an audio cumulative distribution probability G1, a visual cumulative distribution probability G2, a first audio-visual cumulative distribution probability G3, a second audio-visual cumulative distribution probability G4, an estimate value G5, a first subtraction value (promoting effect index) G6 (see Fig. 15), a second subtraction value (promoting effect index) G7 (see Fig. 15), as shown in Fig. 12. The audio cumulative distribution probability G1 is a cumulative distribution probability of the response times to the audio stimulus in the audio selective attention condition. The visual cumulative distribution probability G2 is a cumulative distribution probability of the response times to the visual stimulus in the visual selective attention condition. The first audio-visual cumulative distribution probability G3 is a cumulative distribution probability of the response times to the audio-visual stimulus in the audio selective attention condition. The second audio-visual cumulative distribution probability G4 is a cumulative distribution probability to the audio-visual stimulus in the visual selective attention condition. The estimate value G5 is computed as an estimate value of the cumulative distribution probability of the response times to the audio-visual stimulus on the basis of the audio cumulative distribution probability G1 and the visual cumulative distribution probability. The first subtraction value G6 is obtained by subtracting the estimate value G5 from the first audio-visual cumulative distribution probability G3. The second subtraction value G7 is obtained by subtracting the estimate value G5 from the second audio-visual cumulative distribution probability G4.

Here, the cumulative distribution probability is shown as a rate of the sum total of data of all measured response times, which is sorted into each of segments and exists from the start of the stimulus provision to the segment, to the total number of the data. The segment is configured by dividing a period between the start of the stimulus provision and the end of the response period into a plurality of equal periods (e.g., 10 ms).

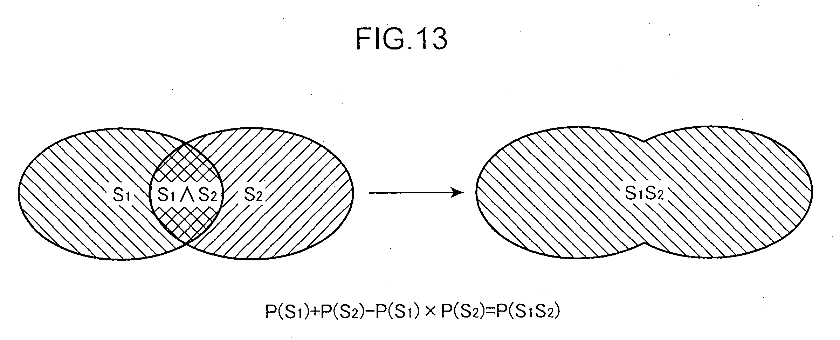

The estimate value G5 can be computed by using a race model, as shown in Fig. 13. Here, the race model is a model for statistically computing the estimate value G5 being logical sum [P(S1S2)] by subtracting logical product [P(S1) x P(S2)] from sum [P(S1) + P(S2)] of the audio cumulative distribution probability G1 and the visual cumulative distribution probability G2.

The first subtraction value G6 or the second subtraction value G7 being an index indicating the degree of the promoting effect by the audio-visual integration is computed by subtracting the thus computed estimate value G5 from the first audio-visual cumulative distribution probability G3 or the second audio-visual cumulative distribution probability G4. That is, the subtraction values G6 and G7 are each an index indicating how fast (slow) a response time in which the audio-visual stimulus is actually provided is, compared to a response time (estimate value G5) to the audio-visual stimulus, which is estimated from the response times in which the audio stimulus and the visual stimulus are individually provided.

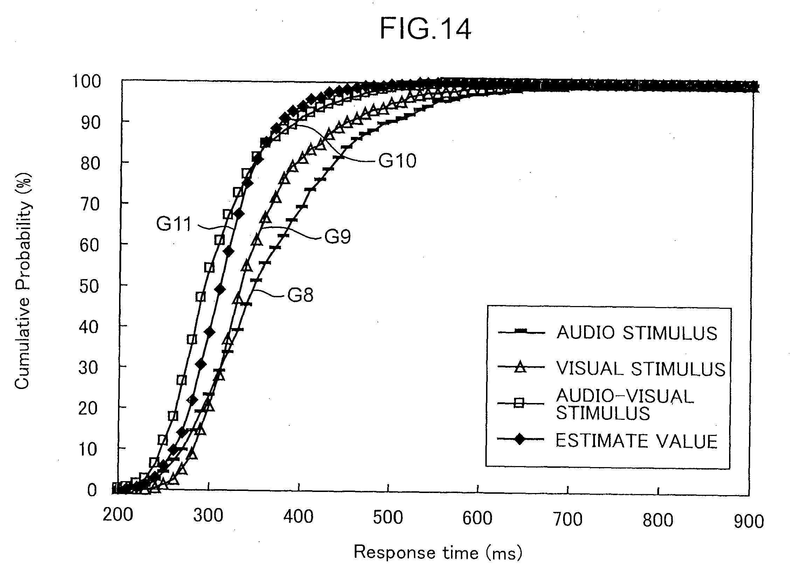

The index computation unit 16 computes a subtraction value (promoting effect index) G12 shown in Fig. 15, on the basis of the response times to the three kinds of stimuli in the audio-visual division attention condition. Specifically, the index computation unit 16 computes an audio cumulative distribution probability G8, a visual cumulative distribution probability G9, an audio-visual cumulative distribution probability G10, an estimate value G11, and the subtraction value G12 (see Fig. 15), as shown in Fig. 14. The audio cumulative distribution probability G8 is a cumulative distribution probability of the response times to the audio stimulus in the audio-visual division attention condition. The visual cumulative distribution probability G9 is a cumulative distribution probability of the response times to the visual stimulus in the audio-visual division attention condition. The audio-visual cumulative distribution probability G10 is a cumulative distribution probability of the response times to the audio-visual stimulus in the audio-visual division attention condition. The estimate value G11 is computed as an estimate value of the cumulative distribution probability of the response times to the audio-visual stimulus, by using the race model on the basis of the audio cumulative distribution probability G8 and the visual cumulative distribution probability G9. The subtraction value G12 is obtained by subtracting the estimate value G11 from the audio-visual cumulative distribution probability G10. The subtraction value G12 is an index indicating the degree of the promoting effect by the audio-visual integration, similarly to the respective subtraction values G6 and G7.

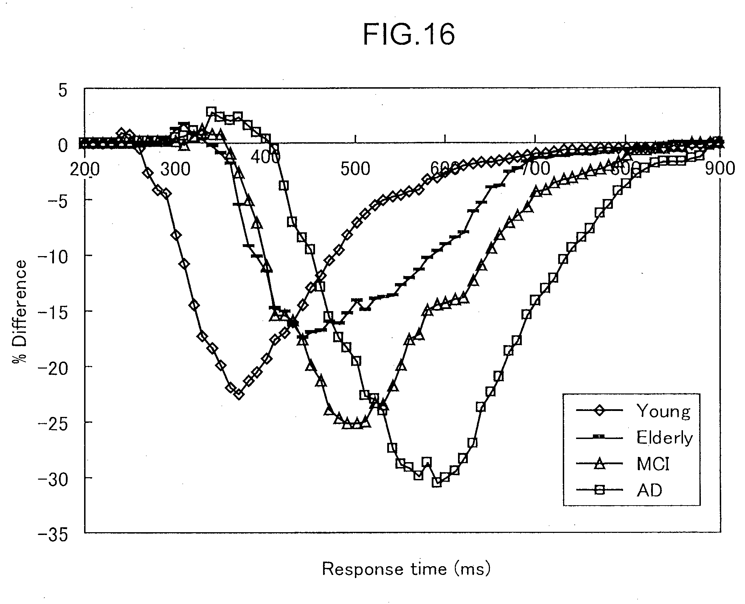

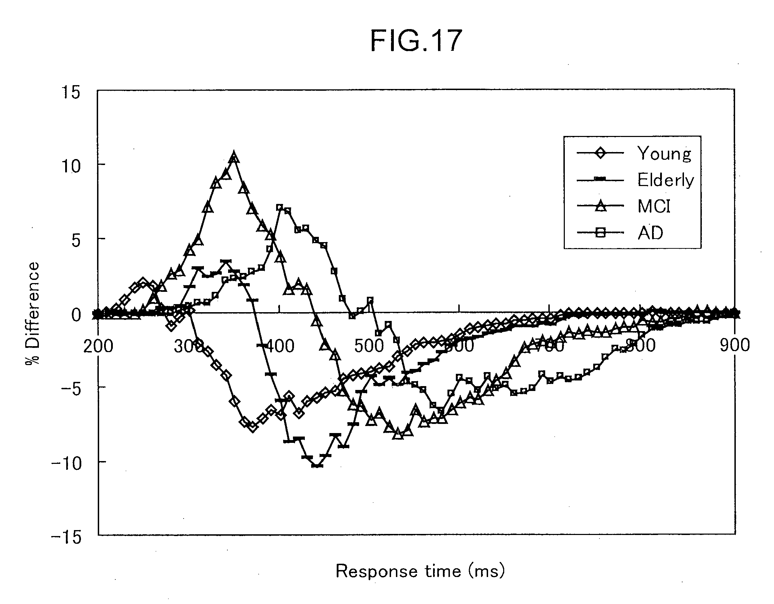

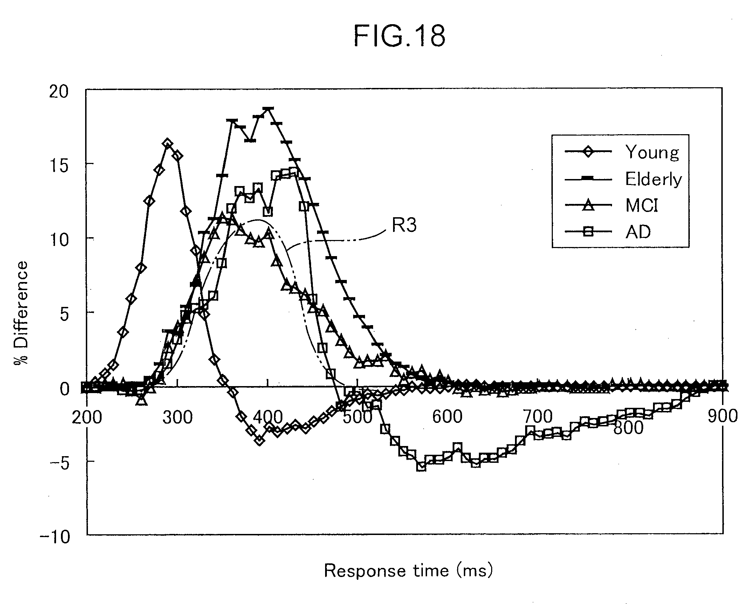

Here, storage unit 8 stores information shown in Fig. 16 to Fig. 18 for comparison with the respective subtraction values G6, G7 and G12 computed by the index computation unit 16. Fig. 16 is a graph showing subtraction values in the audio selective attention condition by group. Fig. 17 is a graph showing subtraction values in the visual selective attention condition by group. Fig. 18 is a graph showing subtraction values in the audio-visual division attention condition by group. A chart showing Young of Fig. 16 corresponds to the subtraction value G6, a chart showing Young of Fig. 17 corresponds to the subtraction value G7, and a chart showing Young of Fig. 18 corresponds to the subtraction value G12.

As shown in Fig. 16 to Fig. 18, significant differences exist between the subtraction values of the respective groups at least in preset response time ranges. For example, it is found that the significant difference exists between the subtraction values of the MCI and Elderly of Fig. 16 at least in a response time range of about 500 ms. It is found that such a significant difference in the prescribed temperature range exists between all groups in all graphs of Fig. 16 to Fig. 18. Accordingly, the group to which the specified subject H belongs can be determined by comparing a subtraction value computed for the specified subject H with the subtraction values shown in Fig. 16 to Fig. 18 in a response time range where significant differences exist. Particularly, distinguishing between Elderly and CMI patients, which is difficult to be accurately determined only with the response times described above, is enabled by utilizing Fig. 16 to Fig. 18.

The data creation unit 17 (see Fig. 2) creates data in which the subtraction value computed for the specified subject H is compared with the subtraction value in the same condition among the subtraction value shown in Fig. 16 to Fig. 18, stored in the storage unit 8. For example, the data creation unit 17 creates a graph in which an average value R3 computed for the specified subject H is shown on a graph of the subtraction values of Fig. 18, and displays this graph on the display 2. Additionally, data creation unit 17 can also produce a graph in which the subtraction value computed for the specified subject H is shown on a graph of the subtraction values of Fig. 16 and Fig. 17. Moreover, the data creation unit 17 can also display all graphs created on the basis of Fig. 16 to Fig. 18 on the display 2.

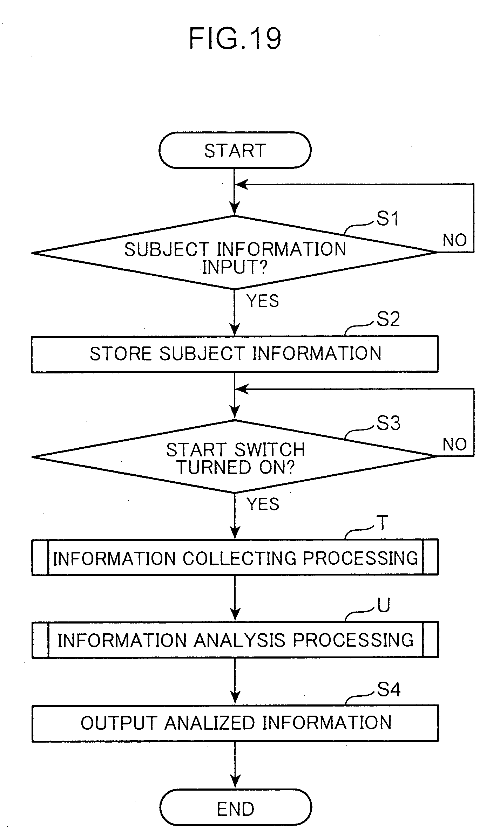

Hereinafter, processes performed by the control unit 5 will be described with reference to Fig. 19 to Fig. 25.

Fig. 19 is a flowchart showing the main routine of the processes performed by the control unit 5 of Fig. 2.

When the control unit 5 starts the process, it is first determined whether or not information (age, sex, etc.) of the subject H is input with an input unit (keyboard, etc.) not shown in the drawing (Step S1). When the subject information is input (YES in Step S1), this subject information is stored in the storage unit 8 (Step S2).

Next, it is determined whether or not a start switch (not shown) is turned on (Step S3). When the start switch is turned on (YES in Step S3), information collecting processing T is performed. The information collected in the information collecting processing T is analyzed in following information analysis processing U. The information analyzed by the information analysis processing U is displayed on the display 2 (Step S4), and the process is terminated.

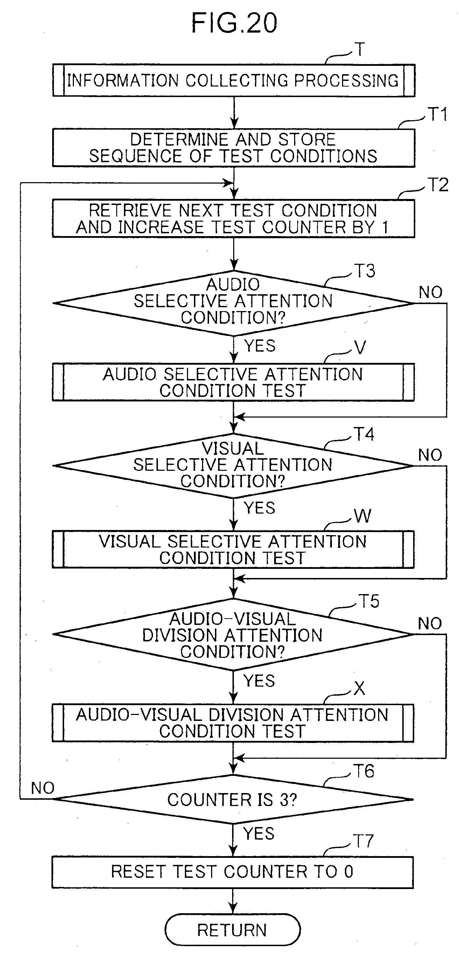

Fig. 20 is a flowchart showing information collecting processing of Fig. 19.

When the information collecting processing T starts, sequence of the three kinds of conditions, namely the audio selective attention condition, the visual selective attention condition, and the audio-visual division attention condition are decided, and stored in the storage unit 8 (Step T1). That is, in this embodiment, tests for providing stimuli in all of the three kinds of conditions are performed.

Next test condition is retrieved from the storage unit 8, and a test counter is increased by 1 (Step T2: selection step). Here, the test counter is a counter counting increased numbers every time one condition among the three kinds of conditions is selected.

It is determined whether or not the retrieved condition is the audio selective attention condition (Step T3). When determining that the retrieved condition is the audio selective attention condition (YES in Step T3), an audio selective attention condition test V is performed, and Step T4 is thereafter performed.

On the other hand, when determining that the retrieved condition is not the audio selective attention condition in Step T3, it is determined whether or not the retrieved condition is the visual selective attention condition (Step T4). When determining that the retrieved condition is the visual selective attention condition in this Step T4, a visual selective attention condition test W is performed, and Step T5 is thereafter performed. On the other hand, when determining that the retrieved condition is not the visual selective attention condition in Step T4, it is determined whether or not the retrieved condition is the audio-visual division attention condition (Step T5).

When determining that the retrieved condition is the audio-visual division attention condition in Step T5, an audio-visual division attention condition test X is performed, and Step T6 is thereafter performed. On the other hand, when determining that the retrieved condition is not the audio-visual division attention condition in Step T5, it is determined whether or not the test counter is 3 (Step T6). That is, in Step T6, it is determined whether or not the tests under all the three kinds of conditions are terminated.

When determining that the test counter is not 3 in Step T6, Step T2 is repeatedly performed. On the other hand, when determining that the test counter is 3, the test counter is reset to 0, and the process is returned to the main routine of Fig. 19.

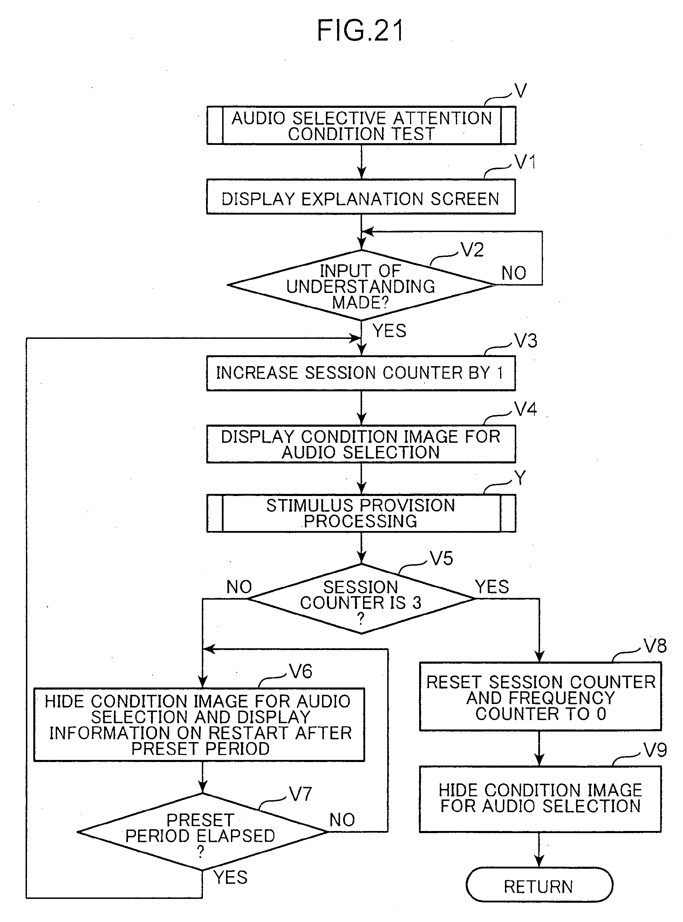

Fig. 21 is a flowchart showing processes of the audio selective attention condition test of Fig. 20.

When the audio selective attention condition test V starts, an explanation screen regarding a test method is displayed (Step V1). This explanation screen includes the following information (1) to (3).

(1) Pay attention only to information obtained through an auditory sense to respond in this test.
(2) Operate the right operation unit 4 when hearing the sound S from the right ear pad unit 3, and operate the left operation unit 4 when hearing the sound S from the left ear pad unit 3.
(3) Operate both of the operation units 4 when understanding (1) and (2).

Next, it is determined whether or not input indicating that the explanation screen is understood is made by the operation units 4 (Step V2). When determining that the input is made, a session counter is increased by 1 (Step V3). Here, the session counter counts the number of times that a stimulus provision processing Y, described later, is performed.

Then, the explanation screen is hidden, and the condition image D1 for audio selection (see Fig. 2A) is displayed on the display 2 (Step V4: offer step), and the stimulus provision processing Y is performed.

When the stimulus provision processing Y is terminated, it is determined whether or not the session counter is 3 (Step V5). That is, in Step V5, it is determined whether or not the stimulus provision processing Y is performed three times.

When determining that the session counter is not 3 in Step V5, the condition image D1 for audio selection is hidden, and information that a test is restart after the preset period is displayed (Step V6). In Step V6, a stimulus frequency counter, described later is reset to 0. Then, the process stands by until the preset period elapses (Step V7). When determining that the preset time has elapsed in Step V7, Step V3 is repeatedly performed.

On the other hand, when determining that the session counter is 3 in Step V5, the session counter, and the stimulus frequency counter, described later, are reset to 0 (Step V8). Then, the condition image D1 for audio selection is hidden (Step V9), and the process is returned to the process of Fig. 20.

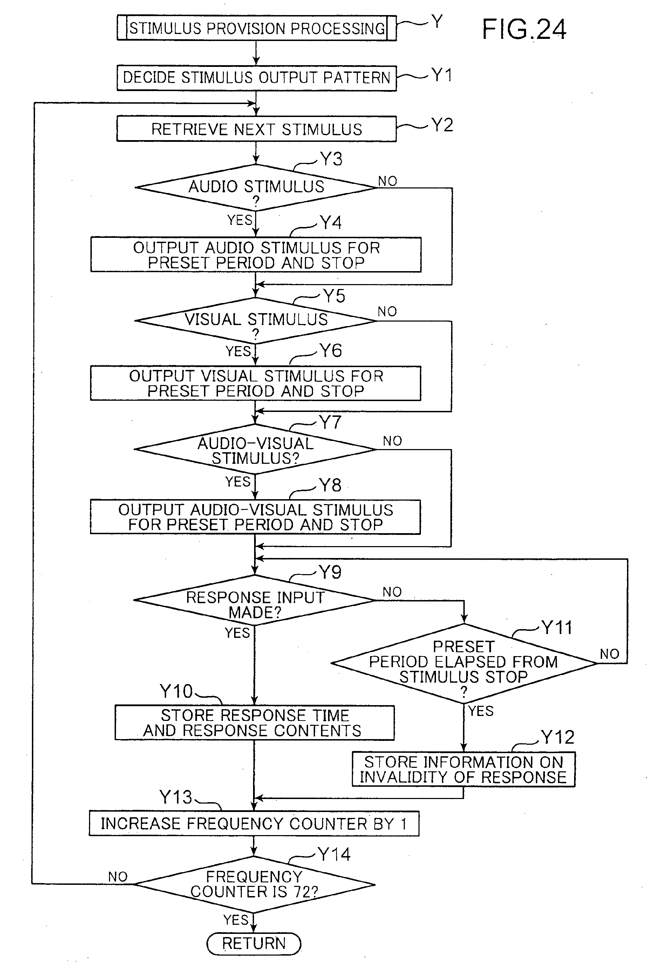

The stimulus provision processing Y of Fig. 21 will be described with reference to Fig. 24.

When the stimulus provision processing Y starts, a stimulus output pattern is decided and stored in the storage unit 8 (Step Y1). Here, the stimulus output pattern is a pattern including 24 audio stimulus times, 24 visual stimulus times, and 24 audio-visual stimulus times. The sequence of the stimuli included in the stimulus output pattern is random.

Next stimulus is retrieved from the storage unit 8 (Step Y2), it is determined whether or not the retrieved stimulus is the audio stimulus (Step Y3).

When determining that the retrieved stimulus is the audio stimulus in Step Y3, the audio stimulus is output for the preset period (e.g., 150 ms) and stopped (Step Y4: stimulus provision step), and Step Y5 is thereafter performed. On the other hand, when determining that the retrieved stimulus is not the audio stimulus in Step Y3, it is determined whether or not the retrieved stimulus is the visual stimulus (Step Y5).

When determining that the retrieved stimulus is the visual stimulus in Step Y5, the visual stimulus is output for the preset period (e.g., 150 ms) and stopped (Step Y6: stimulus provision step), and Step Y7 is thereafter performed. On the other hand, when determining that the retrieved stimulus is not the visual stimulus in Step Y5, it is determined whether or not the retrieved stimulus is the audio-visual stimulus (Step Y7).

When determining that the retrieved stimulus is the audio-visual stimulus in Step Y7, the audio-visual stimulus is output for the preset period (e.g., 150 ms) and stopped (Step Y8: stimulus provision step), and Step Y9 is thereafter performed. On the other hand, when determining that the retrieved stimulus is not the audio-visual stimulus in Step Y7, it is determined whether or not response input by the operation unit 4 is made (Step Y9).

When determining that the response input is made in Step Y9, the response time and response contents are stored in the storage unit 8 (Step Y10: measurement step), the frequency counter is increased by 1 (Step Y13).

On the other hand, when determining that the response input is not made in Step Y9, it is determined whether or not the preset period is stopped from the stimulus stop, that is, whether or not the response period expires (Step Y11). When determining that the response period expires in Step Y 11, the storage unit 8 stores information indicating that response to the stimulus is invalidity (Step Y12), and the frequency counter is increased by 1.

Then, it is determined whether or not the frequency counter is 72 (Step Y14). When determining that the frequency counter is not 72 in Step Y14, Step Y2 is repeatedly performed. On the other hand, when determining that the frequency counter is 72 in Step Y14, the process is returned to the process of Fig. 21.

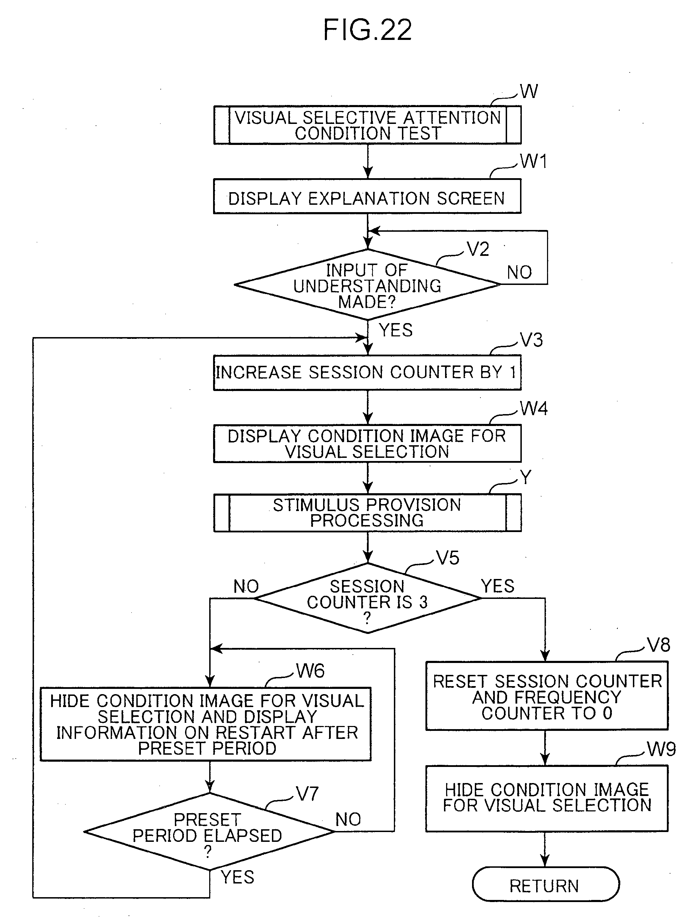

The contents of processes of the visual selective attention condition test of Fig. 20 will be described with reference to Fig. 22. Processes of Fig. 22 similar to the processes of the audio selective attention condition test of Fig. 21 are denoted by the same reference numerals, and description thereof will be omitted.

With reference with Fig. 22, when the visual selective attention condition test W is performed, an explanation screen regarding a test method is displayed (Step W1). This explanation screen includes the following information (1) to (3).

(1) Pay attention only to information obtained through a visual sense to respond in this test.
(2) Operate the right operation unit 4 when the stimulus image D4 is displayed on the right side of the display 2, and operate the left operation unit 4 when the stimulus image D4 is displayed on the left side of the display 2.
(3) Operate both of the operation units 4 when understanding (1) and (2).

Next, after Step V2 and V3 are sequentially performed, the explanation screen is hidden, and the condition image D2 for visual selection (see Fig. 2B) is displayed on the display 2 (Step W4: offer step).

When determining that the session counter is not 3 in Step V5, the condition image D2 for visual selection is hidden, and information that a test is restart after the prescribed period is displayed (Step W6). In Step W6, reset of the frequency counter to 0 is also performed.

Then, after Step V8, the condition image D2 for visual selection is hidden (Step W9), the process is returned to the process of Fig. 20.

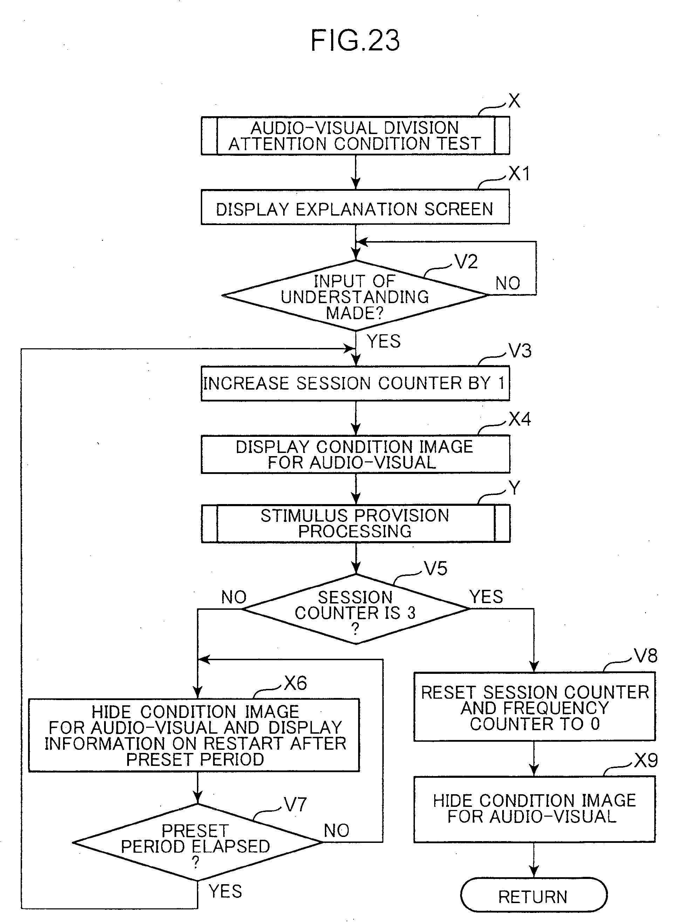

The contents of processes of the audio-visual division attention condition test X of Fig. 20 will be described with reference to Fig. 23. Processes of Fig. 23 similar to the processes of the audio selective attention condition test V of Fig. 21 are denoted by the same reference numerals, and description thereof will be omitted.

With reference to Fig. 23, when the audio-visual division attention condition test X is performed, an explanation screen regarding a test method is displayed (Step X1). This explanation screen includes the following information (1) to (4).

(1) Pay attention only to information obtained through visual and auditory senses to response in this test.

(2) Operate the right operation unit 4 when hearing the sound S from the right ear pad unit 3 and when the stimulus image D4 is displayed on the right side of the display 2.

(3) Operate the left operation unit 4 when hearing the sound S from the left ear pad unit 3 and when the stimulus image D4 is displayed on the left side of the display 2.

(4) Operate both ot the operation units 4 when understanding (1) and (2).

Next, after Step V2 and V3 are sequentially performed, the explanation screen is hidden, and the audio-visual condition image D3 (see Fig. 2C) is displayed on the display 2 (Step X4: offer step).

When determining that the session counter is not 3 in Step V5, the audio-visual condition image D3 is hidden, and information that a test is restart after the prescribed period is displayed (Step X6). In Step X6, reset of the frequency counter to 0 is also performed.

Then, after Step V8, the audio-visual condition image D3 is hidden (Step X9), the process is returned to the process of Fig. 20.

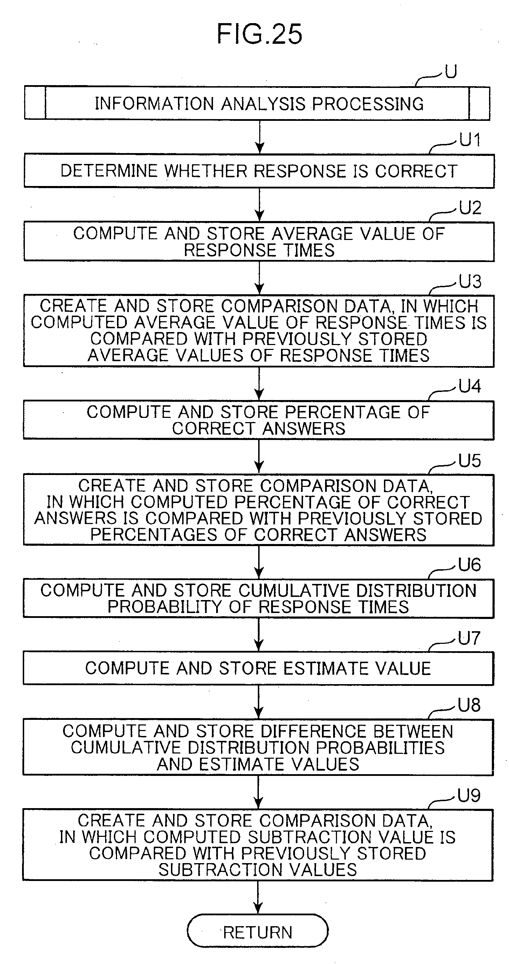

With reference to Fig. 25, the information analysis processing U of Fig. 19 will be described.

When performing the information analysis processing U, it is determined whether responses from the subject H are correct or not (Step U1), an average value of response times is computed to stored in the storage unit 8 (Step U2: average value computation step). Then, the data (e.g., Fig. 8), in which the average value of the response times computed in Step U2 is compared with the reference average values (Fig. 8 and Fig. 9) stored in the storage unit 8 in a preparation step previously performed, is created, and stored in the storage unit 8 (Step U3: comparison step).

A percentage of correct answers in the responses from the subject H is computed (Step U4: correct answer percentage computation step). Then, the data (e.g., Fig. 10), in which the percentage of correct answers computed in Step U4 is compared with the reference percentages of correct answers (Fig. 10 and Fig. 11) stored in the storage unit 8 in the preparation step previously performed, is created, and stored in the storage unit 8 (Step U5: comparison step).

Next, the cumulative distribution probabilities G1 to G4, and G8 to G10 (see Fig. 12 and Fig. 14) are computed on the basis of the response times measured for the subject H, and stored in the storage unit 8 (Step U6). The estimate values G5 and G11 (see Fig. 12 and Fig. 14) are computed on the basis of the cumulative distribution probabilities G1, G2, G8 and G9 computed in Step U6, and stored in the storage unit 8 (Step U7). The subtraction values G6, G7 and G12 (see Fig..15) are computed on the basis of the estimate values G5 and G11 computed in Step U7, and cumulative distribution probabilities G3, G4 and G10, and stored in the storage unit 8 (Step U8: first and second indexes computation step). The comparison data (e.g., Fig. 18), in which the subtraction values G6, G7 and G12 computed in Step U8 are compared with the subtraction values (Fig. 16 to Fig. 18) stored in the storage unit 8 in the preparation step previously performed, is created, and stored in the storage unit 8 (Step U9: comparison step).

As described above, according to the embodiment, the average values (Fig. 8 and Fig. 9) of the response times previously stored for the plurality of subjects H including AD patients is compared with the average value of the response times obtained for the specified subject H, thereby enabling the onset of dementia for the specified subject H to be verified.

Specifically, the significant differences exist between the average values of the response times of the AD patients and people other than the AD patients, as shown in Fig. 9, and hence the previously stored average values of the response times are compared with the average value computed for the specified subject H as in the embodiment, thereby enabling the onset of AD to be verified.

Particularly, in the embodiment, the subject H is required to select one of the two alternatively selectable selection items (right or left), and hence a plurality of subjects can respond at relatively even speed regardless of interest or concern to the provided information, unlike a conventional case where a meaningful object is specified.

Accordingly, according to the embodiment, information (response times) for verifying the onset of dementia can be obtained while suppressing individual differences between the subjects H.

Additionally, as in the embodiment, it is possible to distinguish between MCI patients and healthy elderly people by comparing the values (see Fig. 15) previously prepared, which are the subtraction values as indexes indicating the degrees of the promoting effects by the audio-visual integration, with the values (Fig. 16 to Fig. 18) computed for the specified subject H. Specifically, the promoting effect by the audio-visual integration has a property of showing an higher effect with increase in person ages, and reducing with reduction in brain functions, and hence it is possible to distinguish between the healthy elderly people and the MCI patients by comparison of the promoting effects.

Furthermore, according to the embodiment, the previously prepared reference percentages of correct answers (Fig. 10 and Fig. 11) are compared with the percentage of correct answers computed for the specified subject H by utilizing the property (see Fig. 11) of the percentage of correct answers, in which the significant differences are found between the AD patients and people other than the AD patients, thereby enabling the onset of AD to be verified.

In the embodiment, the data (Fig. 8, Fig. 10 and Fig. 18), in which the values measured for the specified subject H is compared with the values stored in the storage unit 8, is displayed on the display 2. Accordingly, a healthcare worker who sees the display 2 can determine the onset of dementia.

In the embodiment, as shown in Fig. 3, the condition image D1 showing the audio selective attention condition, the condition image D2 showing the visual selective attention condition, or the condition image D3 showing the audio-visual division attention condition are displayed. Accordingly, the subject H can visually recognize which condition is selected among the three kinds of conditions.

The verification device 1 according to the embodiment displays the data (Fig. 8, Fig. 10 and Fig. 18), in which the values measured for the specified subject H is compared with the values stored in the storage unit 8 on the display 2, but is not limited to this. The verification device 1 may determine which group (AD, CMI, Elderly or Young) the specified subject H belongs to, and the determination result may be displayed on the display 2.

Specifically, a threshold value for differentiating the average values of the response times of the AD patients from the average values of the response times of the people other than the AD patients can be previously prepared on the basis of the average values of the response times (see Fig. 9) stored in the storage unit 8, and be stored in the storage unit 8. Then, the data creation unit 17 can determine whether or not the specified subject H is an AD patient by comparing the average value of the response times computed for the specified subject H with the threshold value, and display the determination result on the display 2.

While an example of preparing the threshold value of the response time has been explained, the threshold value of the percentage of correct answers can also be prepared on the basis of the percentages of correct answers shown in Fig. 11. Consequently, the data creation unit 17 determines whether or not the specified subject H is an AD patient, and displays the result on the display 2.

Additionally, it is possible to previously prepare threshold values for differentiating the subtraction values of the four groups, namely, AD, MCI, Elderly and Young on the basis of the subtraction values (Fig. 16 to Fig. 18) which are promoting effect indexes and stored in the storage unit 8, and to store these threshold values in the storage unit 8. Then, the data creation unit 17 can determine which group the specified subject H belongs to among the four groups by comparing the subtraction value computed for the specified subject H with the threshold values, and display the determination result on the display 2.

The determination as to which group the specified subject H belongs to, can be made by utilizing not only one of Fig. 16 to Fig. 18 but also all of Fig. 16 to Fig. 18. Specifically, three determination results can be obtained by comparing the three subtraction values in the three kinds of conditions, measured for the specified subject H, with the respective set threshold values of Fig. 16 to Fig. 18. That is, the determination result obtained by comparing the subtraction value in the audio selective condition with the threshold value can be obtained by comparison using Fig. 16. Additionally, the determination result obtained by comparing the subtraction value in the visual selective condition with the threshold value can be obtained by comparison using Fig. 17. Furthermore, the determination result obtained by comparing the subtraction value in the audio-visual division attention condition with the threshold value can be obtained by comparison using Fig. 18. Consequently, the group to which the subject H belongs and the possibility thereof can be determined on the basis of these three determination results.

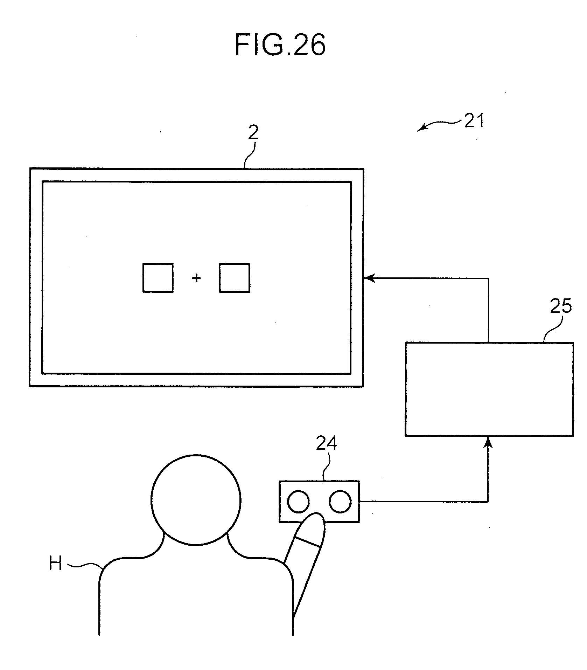

Hereinafter, a device 21 for verifying onset of dementia according to a second embodiment of the present invention will be described with reference to Fig. 26 to Fig. 35. Configurations identical with those of the first embodiment will be denoted by the same reference numerals, and description thereof will be omitted.

With reference to Fig. 26, the verification device 21 includes the display (image display device: visual information provision unit) 2, an operation unit 24 outputting a command in accordance with operation from a subject H, and a control unit 25 electrically connected to the display 2 and the operation unit 24.

The operation unit 24 has a pair of right and left operation buttons. While the operation unit 24 outputs an electric signal indicating a response of "right" by pressing the right button, the operation unit 24 outputs an electric signal indicating a response of "left" by pressing the left button. The operation unit 24 may be replaced by, for example, a mouse being an input interface of a personal computer.

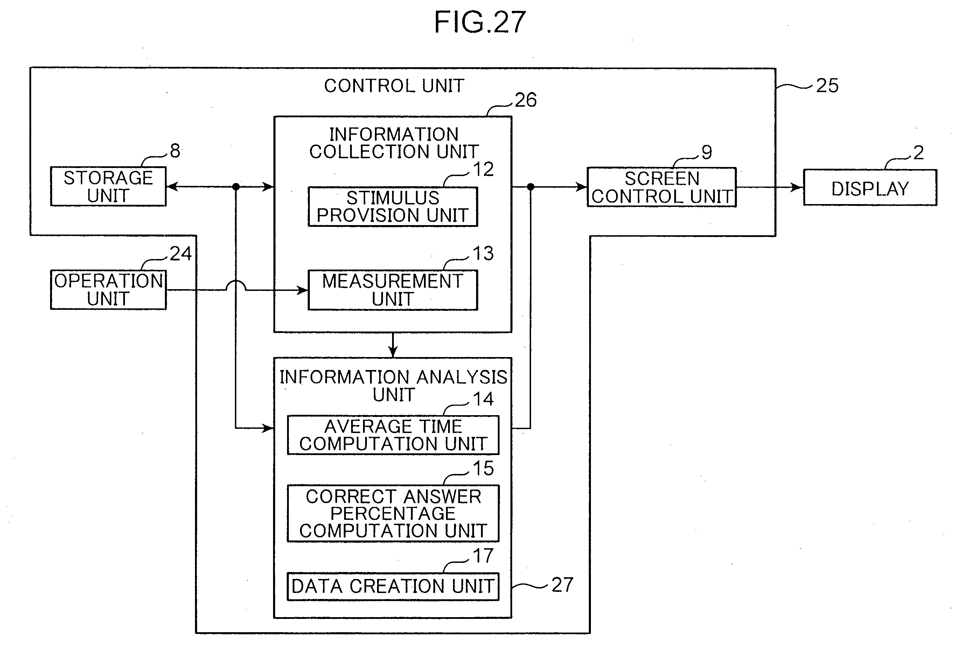

With reference to Fig. 27, the control unit 25 includes an information collection unit 26 for collecting information for verifying existence or non-existence of dementia, an information analysis unit 27 which analyzes the information collected by the information collection unit 26, a storage unit 8 which stores information compared with the information analyzed by the information analysis unit 27, a screen control unit 9 which controls the image display by the display 2 in accordance with commands from the information collection unit 26 and the information analysis unit 27.

The information collection unit 26 provides visual information through the display 2, and receives a response (command from the operation unit 24) from the subject H to this information provision. Specifically, the information collection unit 26 includes the stimulus provision unit 12, and the measurement unit 13.

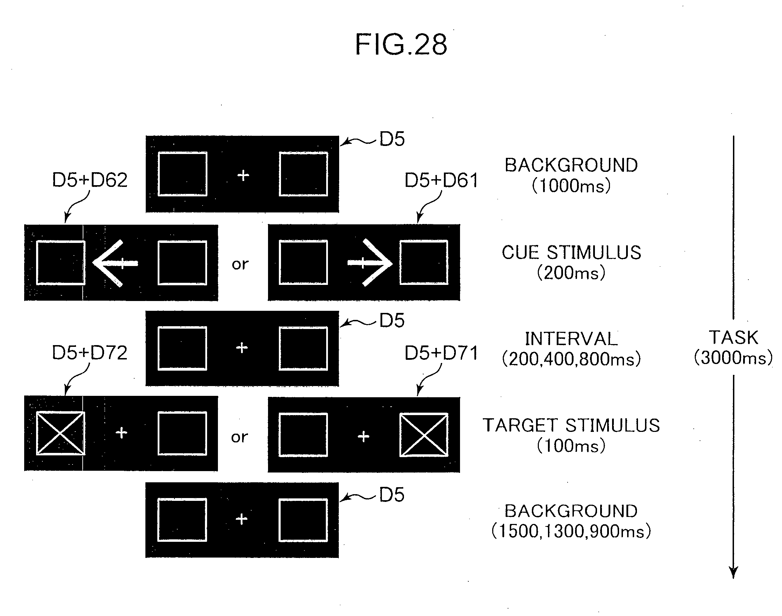

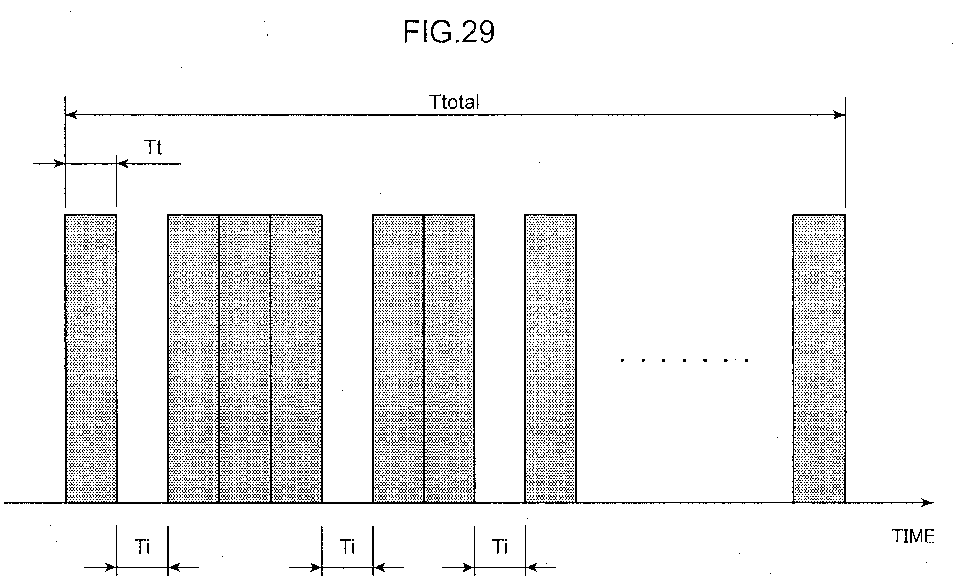

The stimulus provision unit 12 provides the subject H with a visual stimulus through the display 2. Specifically, the stimulus provision unit 12 provides the subject H with tasks shown in Fig. 28 at intervals Ti between the tasks a plurality of times appropriately, as shown in Fig. 29.

More specifically, the stimulus provision unit 12 performs the tasks shown in Fig. 28 with respect to the one subject H 90 times. In this embodiment, the intervals of periods Ti (3000 ms) are randomly inserted between the respective tasks, as shown in Fig. 29. The interval is inserted 30 times while the tasks are performed 90 times. Therefore, in this embodiment, a case where the interval is provided between the continuous two tasks, and a case where no interval is provided between the continuous two tasks exist.

The time required for each task is 3000 ms, as shown in Fig. 28. As a result, a test time Ttotal for each subject H is 6 min.

Specifically, the task includes a background period (1000 ms), a cue stimulus period (200 ms), an interval period (200 ms, 400 ms, 800 ms), a target stimulus period (100 ms), and a background period (1500 ms, 1300 ms, 900 ms) in this order. When the interval period is 200 ms, the following background period is 1500 ms. When the interval period is 400 ms, the following background period is 1300 ms. When the interval period is 800 ms, the following background period is 900 ms. Among the total number of the performed tasks (90 times), 1/3 of the tasks have an interval period of 200 ms, 1/3 of the tasks have an interval period of 400 ms, 1/3 of the tasks have an interval period of 800 ms. The sequence of the respective interval periods is randomly set.

In the background period, the stimulus provision unit 12 displays only a background image D5. The background image D5 has a pair of square images arranged left and right at an interval therebetween, and a "+" image arranged between these square images. In the cue stimulus period, the stimulus provision unit 12 displays a cue image (cue stimulus) D61 or a cue image (cue stimulus) D62 on the "+" image of the background image D5. The cue image D61 is a right-pointing arrow, and the cue image D62 is a left-pointing arrow. In the interval period, the stimulus provision unit 12 displays only the background image D5. In the target period, the stimulus provision unit 12 displays a target image (target stimulus) D71 or a target image (target stimulus) D72 in addition to the background image D5. The target image D71 is an "x" image displayed inside the right square image of the background image D5. The target image D72 is an "x" image displayed inside the left square image of the background image D5. That is, the target image D71 requires the response of "right" to the subject H, the target image D72 requires the response of "left" to the subject H.

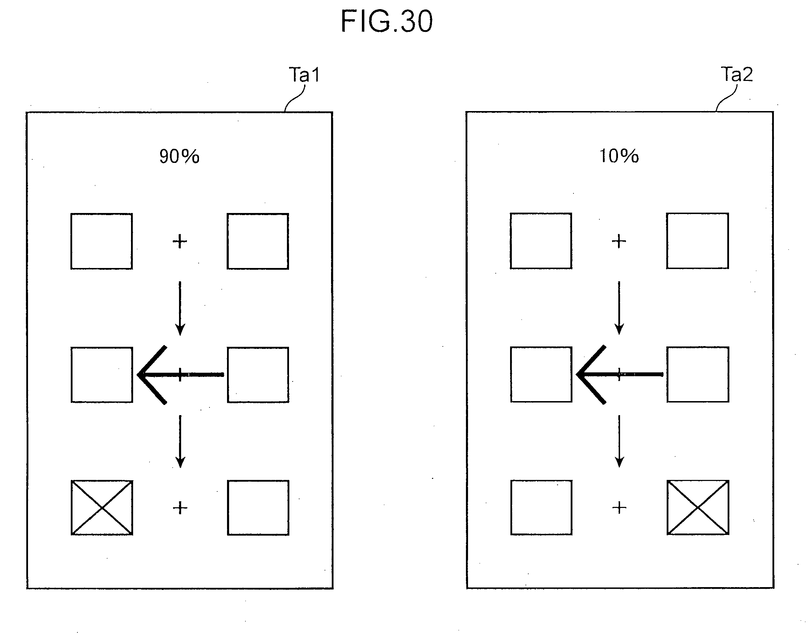

Furthermore, the stimulus provision unit 12 displays the cue images D61 and D62, and the target images D71 and D72 in accordance with a first combination Ta1 or a second combination Ta2 shown in Fig. 30. The first combination Ta1 is a combination in which pointing directions of the cue images D61 and D62 match positions of the target images D71 and D72. In Fig. 30, while the combination of the left-pointing cue image D62 and the target image D72 located left is disclosed, the first combination Ta1 also includes a combination of the right-pointing cue image D61 and the image D71 located right. The second combination Ta2 is a combination in which the pointing directions of the cue images D61 and D62 are opposite to the positions of the target images D71 and D72. In Fig. 30, while the combination of the left-pointing cue image D62 and the target image D71 located right are disclosed, the second combination Ta2 also includes a combination of the right-pointing cue image D61 and the image D72 located left. Among the tasks performed for each subject H a plurality of times (90 times in this embodiment), 90% (81 times in this embodiment) of the tasks have the first combinations Ta1, and 10% (9 times in this embodiment) of the tasks have the second combinations Ta2. The cue images D61 and D62 included in the first combination Ta1 each correspond to a validity cue stimulus, and the cue images D61 and D62 included in the second combination Ta2 each correspond to an invalidity cue stimulus.

Referring to Fig. 2 again, the measurement unit 13 measures a response time between display start of the target images D71 and D72 and completion of response by the subject H.

The information analysis unit 27 computes information necessary for verifying onset of dementia on the basis of the response times obtained by the information collection unit 26, and compares the computed information with information stored in the storage unit 8.

Specifically, information analysis unit 27 includes an average time computation unit 14 which computes an average value of the response times, a correct answer percentage computation unit 15 which computes a percentage of correct answers in the responses from the subject H, and a data creation unit 17 which creates data in which information computed by the respective computation units 14 and 15 is compared with the information stored in the storage unit 8.

The average time computation unit 14 computes the average value of the response times for each of interval periods 200 ms, 400 ms and 800 ms between the cue images D61 and D62 and the target images D71 and D72. Specifically, the average time computation unit 14 excludes the following [1] to [3] when computing the average value of the response times.

[1] A subject, whose percentage of correct answers is equal to or more than 70%, the percentage of correct answers being computed by the correct answer percentage computation unit described later.
[2] A response time for the second combination Ta2.
[3] An incorrect response (including a case where the both buttons of the operation unit 24 are operated).

Additionally, the average time computation unit 14 can exclude a response time falling under formula (1) and formula (2) from the response times for computing the average values, similarly to the embodiment.

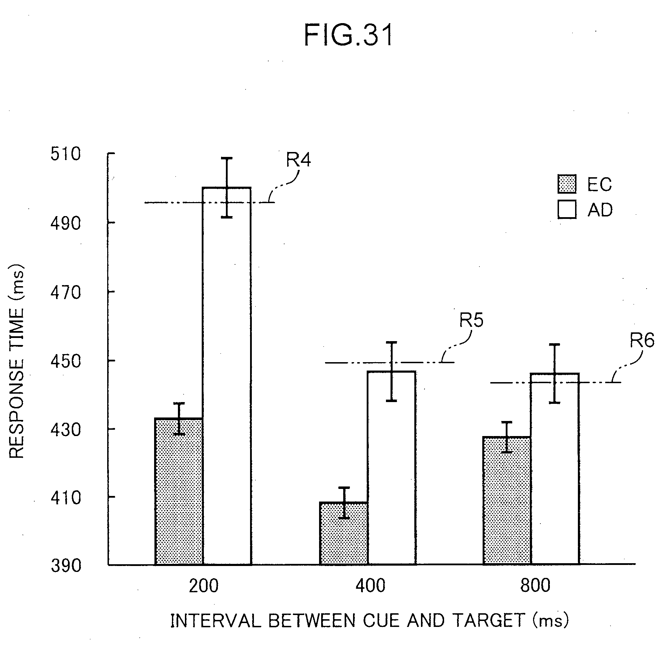

Here, the storage unit 8 stores information shown in Fig. 31 for comparison with the average values computed by the average time computation unit 14. Specifically, the storage unit 8 stores reference average values being average values of response times previously computed for a plurality of subjects H belonging to two groups including dementia patients (denoted by AD) and healthy elderly people (denoted by EC (Elderly Control)) (preparation step), as shown in Fig. 31. The reference average values are computed by the average time computation unit 14. Additionally, the reference average values are computed for respective intervals between the cue images D61 and D62 and the target images D71 and D72 (cue-target intervals) of 200 ms, 400 ms and 800 ms. Respective significant differences exist between AD 200 ms and EC 200 ms, between AD 400 ms and EC 400 ms, and between AD 800 ms and EC 800 ms at P value < 0.05. Furthermore, respective significant differences exist between AD 200 ms and EC 200 ms, and between AD 400 ms and EC 400 ms, also at P value < 0.001.

It is considered that the reason why the significant difference thus exists is "attention" paid when a human visually collects information from outside. Specifically, the "attention" includes passive attention which is paid when reflexively turning attention to sudden change of an uncontemplated stimulus, and active attention which is paid when consciously turning attention to a certain position. Here, in the embodiment, the active attention of the subject H is paid by instructing one of two alternatively selectable selection items by the cue images D61 and D62. It is considered that people other than the dementia patients have higher ability for introducing an item indicated by the target stimulus with this active attention paid than dementia patients, and hence the significant difference exists.

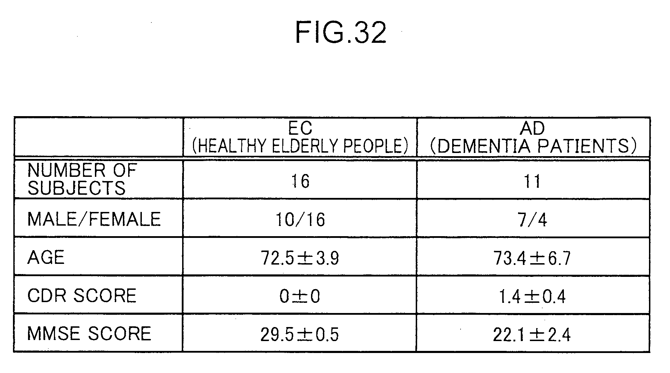

The reference average values shown in Fig. 31 are computed for the subjects H shown in Fig. 32. Here, a CDR score is abbreviation of a Clinical Dementia Rating score. An MMSE score is abbreviation of a Mini-Mental State Examination score. These CDR score and MMSE score are each an index of providing an indication of the degree of dementia.

The data creation unit 17 (see Fig. 2) creates data in which the average value of the response times computed for the specified subject H is compared with the reference average values (see Fig. 31) stored in the storage unit 8. Specifically, as shown in Fig. 31, the data creation unit 17 creates a graph in which average values R4 to R6 of the response times computed for the specified subjects H are shown on a graph of the reference average values, and displays this graph on the display 2. The average value R4 is an average value of response times of a cue-target interval of 200 ms. The average value R5 is an average value of response times of a cue-target interval of 400 ms. The average value R6 is an average value of response times of a cue-target interval of 800 ms.

The correct answer percentage computation unit 15 (see Fig. 2) computes a percentage of correct answers for each of the cue-target interval period. The percentages of correct answers computed by the correct answer percentage computation unit 15 are used when the average time computation unit 14 computes the average values and the reference average values.

Hereinafter, processes performed by the control unit 25 will be described with reference to Fig. 19, Fig. 33 to Fig. 35.

Main routine performed by the control unit 25 is similar to that shown in Fig. 19. In this embodiment, the contents of information collecting processing T and information analysis processing U in Fig. 19 are different from those of the embodiment. Accordingly, the information collecting processing T and the information analysis processing U according to this embodiment will be described.

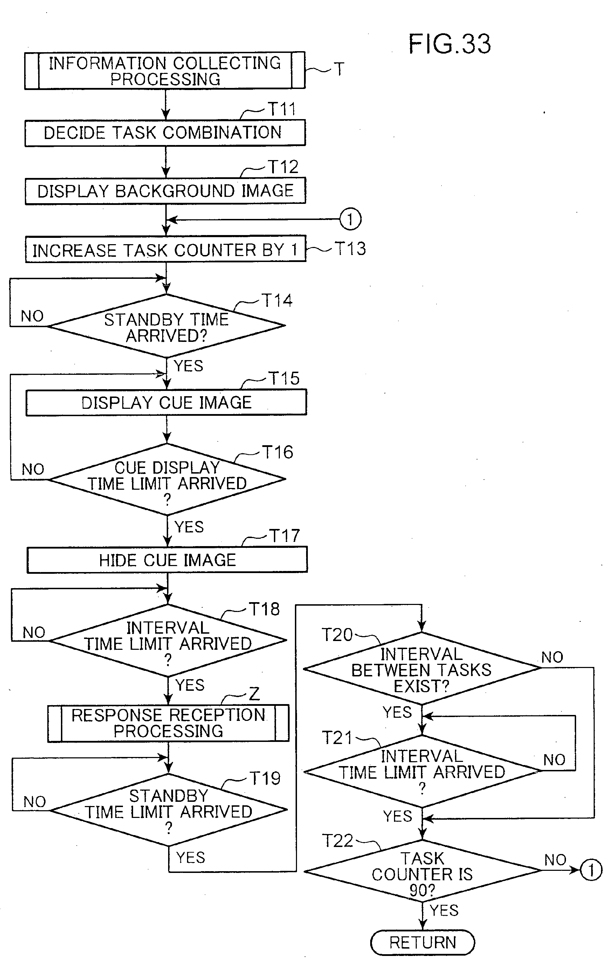

With reference to Fig. 33, when the information collecting processing T starts, the combination of the tasks is decided (Step T11). Specifically, in Step T11, it is determined that the sequence of the tasks performed at 90 times, timing for insertion of the intervals between the respective tasks is decided. The combination of the tasks may be previously stored in the storage unit 8 and retrieved from the storage unit 8 in the Step T11.

As shown in Fig. 28, the background image D5 is displayed on the display 2 (Step T12), and a task counter is increased by 1 (Step T13). Then, it is determined whether or not a standby time preset as the display time of the background image D5 has arrived (Step T14). When determining that the standby time has arrived, the cue image D61 or D62 is displayed in addition to the background image D5 (Step T15: stimulus provision step).

When displaying the cue image D61 or D62, it is determined whether or not a cue display time limit preset as the display time has arrived (Step T16). The background image D5 and the cue image D61 or D62 are displayed until the cue display time limit arrives (during NO in Step T16). On the other hand, when the cue display time limit has arrived (YES in Step T16), the cue image is hidden (Step T17).

Then, it is determined whether or not a cue-target interval time limit has arrived (Step T18). That is, only the background image D5 is displayed within the interval time limit, and when determining that the interval time limit has arrived, response reception processing Z is performed.

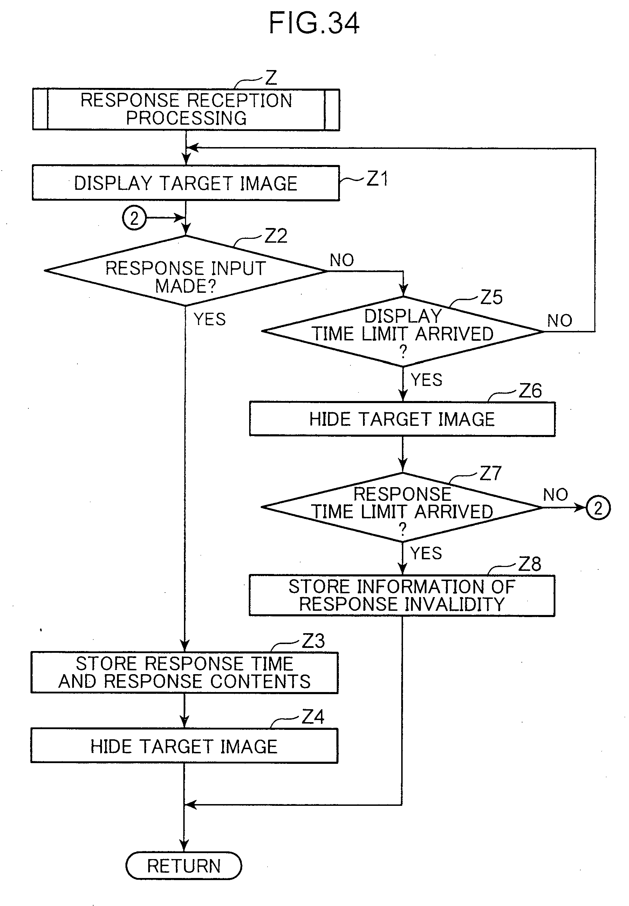

As shown in Fig. 34, when the response reception processing Z is performed, the target image D71 or D72 is displayed in addition to the background image D5 (Step Z1: stimulus provision step).

When the target image D71 or D72 is displayed, it is determined whether or not a response to the target image D71 or D72 by the subject H is input (Step Z2). Here, when determining that the response is input, the response time and the response contents (right or left) are stored (Step Z4: measurement step), the target image D71 or D72 is hidden, and the process is returned to the process of Fig. 33.

On the other hand, when determining that the response is not input in Step Z2, it is determined whether or not a display time limit preset as a display time of the target images D71 and D72 has arrived (Step Z5). Here, when determining that the display time limit has not arrived, Step Z1 is repeatedly performed. On the other hand, when determining that the display time limit has arrived, the target images D71 and D72 are hidden (Step Z6).

Then, it is determined whether or not a response time limit preset as a time for the response of the subject H from display start of the target images D71 and D72 has arrived (Step Z7). Here, when determining that the response time limit has not arrived, Step Z2 is repeatedly performed. On the other hand, when determining that the response time limit has arrived, information that the response by the subject H is invalidity is stored, the process is returned to the process of Fig. 33.

With reference to Fig. 33, when response reception processing is terminated, it is determined whether or not a time limit (standby time limit) of a background period after elapse of the target stimulus period shown in Fig. 28 has arrived (Step T19).

When the standby time limit has arrived in Step T19, it is determined whether or not the interval between the tasks, denoted by symbol Ti in Fig. 29, exists (Step T20). Here, when determining that the interval does not exist, Step T22 is performed. On the other hand, when determining that the interval exists, the arrival of the time limit is awaited (Step T21), and Step T22 is performed.

In Step T22, it is determined whether or not the task counter is 90. Here, when determining that the task counter is not 90 (NO in Step T22), the Step T13 is performed. On the other hand, when determining that the task counter is 90, the process is returned to the main routine shown in Fig. 19.

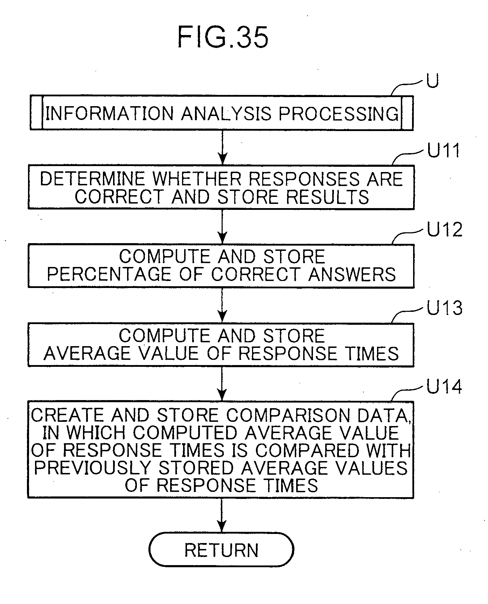

With reference to Fig. 35, when the information analysis processing U is performed, it is determined whether or not the responses from the subject H are correct, and the determination results are stored in the storage unit 8 (Step U11), and a percentage of correct answers is computed and stored in the storage unit 8 (Step U12).

Then, the average value of the response times is computed and stored in the storage unit 8 (Step U13: average value computation step).

Comparison data shown in Fig. 31, in which the average value computed in Step U13 is compared with the reference average values stored in the storage unit 8, is created (Step U14: comparison step), and the process is returned to the main routine shown in Fig. 19. Specifically, in Step U14, the comparison data, in which the average values R4 to R6 of the response times of the subject H measured at this time is superimposed on the graph of the reference average values shown in Fig. 31, is created. This comparison data is displayed on the display 2 in Step S4 of Fig. 19. Therefore, a doctor seeing this comparison data can determine the onset of dementia for the subject H.

As described above, in the embodiment, the target images D71 and D72 are provided after providing the cue images D61 and D62, and hence more valuable information for verifying the onset of dementia can be collected. Specifically, in the embodiment, active attention of the subject H can be paid by instructing one of the two alternatively selectable selection items by the cue images D61 and D62. Then, it is possible to obtain the response time showing the level of the ability for introducing the item indicated by the target images D71 and D72 with this active attention paid.

Accordingly, the onset of dementia can be reliably verified by comparing the average values of the response times measured for the specified subject H with the reference average value.

In the embodiment, the first combination Ta1 and the second combination Ta2 exist as the combination of the cue images D61 and D62 and the target images D71 and D72. Therefore, the subject H can be inhibited from responding on the basis of only the cue images D61 and D62 without paying attention to the target images D71 and D72. The response time corresponding to the ability for introducing the item indicated by the target images D71 and D72 with this active attention paid can be reliably obtained by excluding the response times to the second combination Ta2 from an object to be compared.

In the embodiment, the intervals of 200 ms, 400 ms, and 800 ms are provided between the cue images D61 and D62 and the target images D71 and D72. Consequently, the standby time for the target images D71 and D72 can be given to the subject H with the active attention paid, unlikely to a case of continuously providing the cue images D61 and D62 and the target images D71 and D72. Accordingly, it is possible to suppress variation in the response times by confusion of the cue images D61 and D62 and the target images D71 and D72.

In the embodiment, the response times by the subjects H whose percentage of correct answers is equal to or more than 70% are used as the reference average value. Consequently, the reference average value more accurately indicates the response time to the target images D71 and D72 with the active attention paid. Specifically, the target images D71 and D72 indicate the two alternatively selectable selection items, and hence the percentage of correct answers is 50% even when the response is made without verifying the target images D71 and D72. Therefore, as in the embodiment, the response times, for which the percentage of correct answers is equal to or more than 70%, are extracted, thereby enabling computation of the reference average value based on the response times in which the subject is likely to be made while verifying the target images D71 and D72 to responce.

The verification device 21 displays, on the display 2, the data (Fig. 31), in which the value measured for the specified subject H is compared with the values stored in the storage unit 8, but is not limited to this. The verification device 21 may determine which group (AD or EC) the specified subject H belongs to, and the determination result may be displayed on the display 2.

Specifically, a threshold value for differentiating AD from EC patients can be previously prepared on the basis of the average values (Fig. 31) of the response times stored in the storage unit 8, and be stored in the storage unit 8. Then, the data creation unit 17 can determine whether the specified subject H is AD or EC by comparing the average value of the response times computed for the specified subject H with the threshold value, and display the determination result on the display 2.

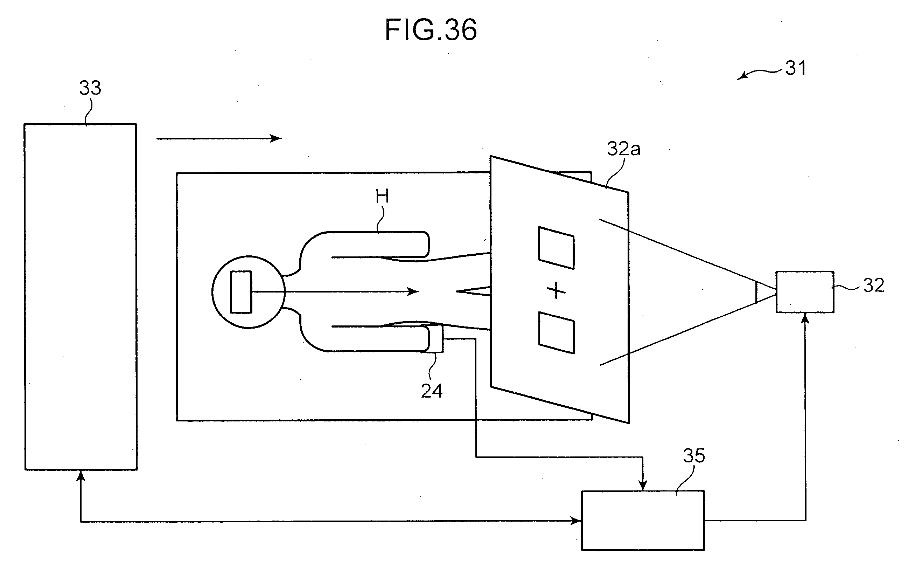

Hereinafter, a modification of the second embodiment will be described with reference to Fig. 36 to Fig. 44. Configurations identical with those of the embodiment will be denoted by the same reference numerals, and description thereof will be omitted.

With reference to Fig. 36, a verification device 31 analyzes change in an active state of a brain of a subject on the basis of a detection signal of an fMRI, while performing a test using the cue images D61 and D62 and the target images D71 and D72 in the second embodiment.

Specifically, the verification device 31 includes a projector 32 (image display device: visual information provision unit) capable of displaying an image to a paper screen 32a, the operation unit 24, an fMRI (functional Magnetic Resonance Imaging) 33 for detecting change of an active state of a brain of a subject H, and a control unit 35 electrically connected to the projector 32, the operation unit 24 and the fMRI 33. In Fig. 36, a state where the fMRI 33 is separated from the subject H is disclosed, but the fMRI 33 is arranged so as to cover a head of the subject H during the test.

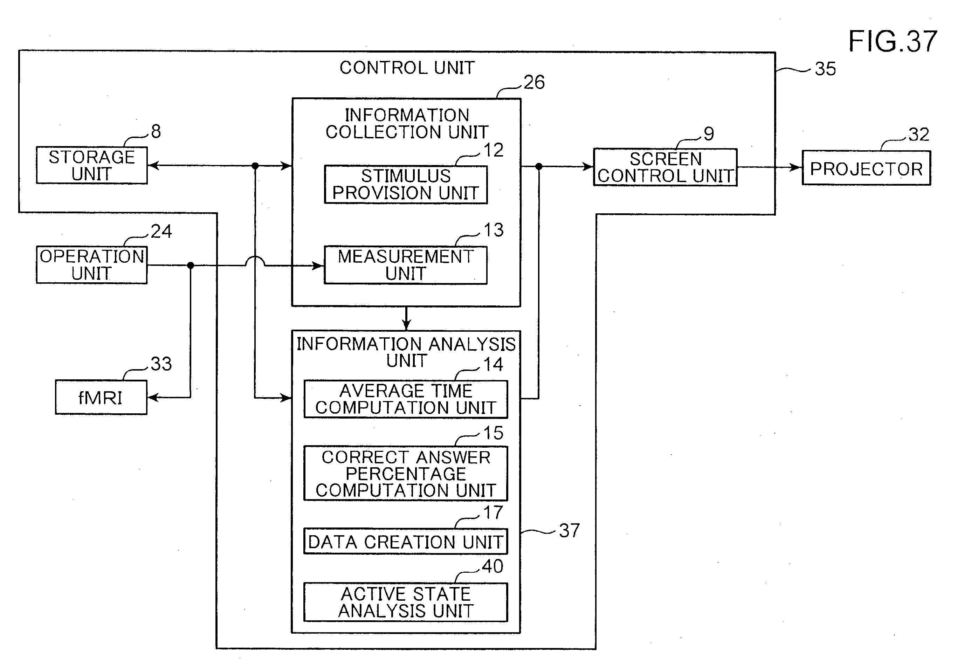

with reference to Fig. 37, the control unit 35 includes an information collection unit 26 for collecting information for verifying existence or non-existence of dementia, an information analysis unit 37 which analyzes the information collected by the information collection unit 26, and a screen control unit 9 which controls the image display by the projector 32 in accordance with commands from the information collection unit 26 and the information analysis unit 37.

The information collection unit 26 provides visual information through the projector 32, and receives a response from the subject H to this information provision. Specifically, the information collection unit 26 includes the stimulus provision unit 12 and the measurement unit 13.

The measurement unit 13 measures a response time between display start of target images D71 and D72 and completion of response by the subject H, similarly to the embodiment. Furthermore, the measurement unit 13 in this embodiment acquires detection values by the fMRI 33 in an execution period between display start of a cue image D61 and reception of a response by the operation unit 24, and in a period (period of an interval Ti of Fig. 29) other than the execution period.

The information analysis unit 37 computes information necessary for verifying onset of dementia on the basis of the response time obtained by the information collection unit 26 and a detection unit of the fMRI 33, and compares the computed information with information stored in the storage unit 8.

Specifically, the information analysis unit 37 includes an average time computation unit 14, a correct answer percentage computation unit 15, an active state analysis unit 40 which analyzes the change of the active state of the brain of the subject H, a data creation unit 17 which creates data in which information analyzed by the active state analysis unit 40 is compared with the information stored in the storage unit 8.

The active state analysis unit 40 analyzes the change of the active state of the brain of the subject H on the basis of the detection value by the fMRI 33 which is acquired by the measurement unit 13. Specifically, the active state analysis unit 40 computes a change rate (hereinafter referred to as a BOLD signal change rate) between MRI signals detected during the execution period between the display start of the cue images D61 and D62 and the reception of the response by the operation unit 24, and MRI signals detected during the period of the interval Ti of Fig. 29.

The BOLD signal change rate indicates the change in the active state of the brain between the execution period in which a stimulus is applied to the brain, and the period of the interval Ti. Specifically, when the stimulus is applied to the brain (at the time of activation), the amount of deoxyhemoglobin (hereinafter referred to as Deoxy-Hb) in the brain increases with increase in oxygen consumption of the brain, and the amount of oxyhemoglobin (hereinafter referred to as Oxy-Hb) increases with increase in blood flow of the brain. More specifically, the increased amount of Oxy-Hb being a diamagnetic substance is extremely large relative to the increase in Deoxy-Hb being a paramagnetic substance. Consequently, so-called BOLD (Blood Oxygenation Level Dependent) effect involving enhancement of the MRI signals and extension of relaxation time of MRI signals is produced. Accordingly, the change rate of this BOLD effect is computed, thereby enabling the change in the active state of the brain between the time of the activation and the time other than the activation to be analyzed.

The active state analysis unit 40 excludes a detection value by the fMRI 33 during a test performed for the second combination Ta2 (see Fig. 30), when computing the BOLD signal change rate. Consequently, it is possible to obtain information on the change of the active state of the brain of the subject H who is provided with a proper cue stimulus (validity cue stimulus) and paying the active attention.

Thus, the active state analysis unit 40 computes the BOLD signal change rate, so that the change of the active state of the brain of the subject H is analyzed.

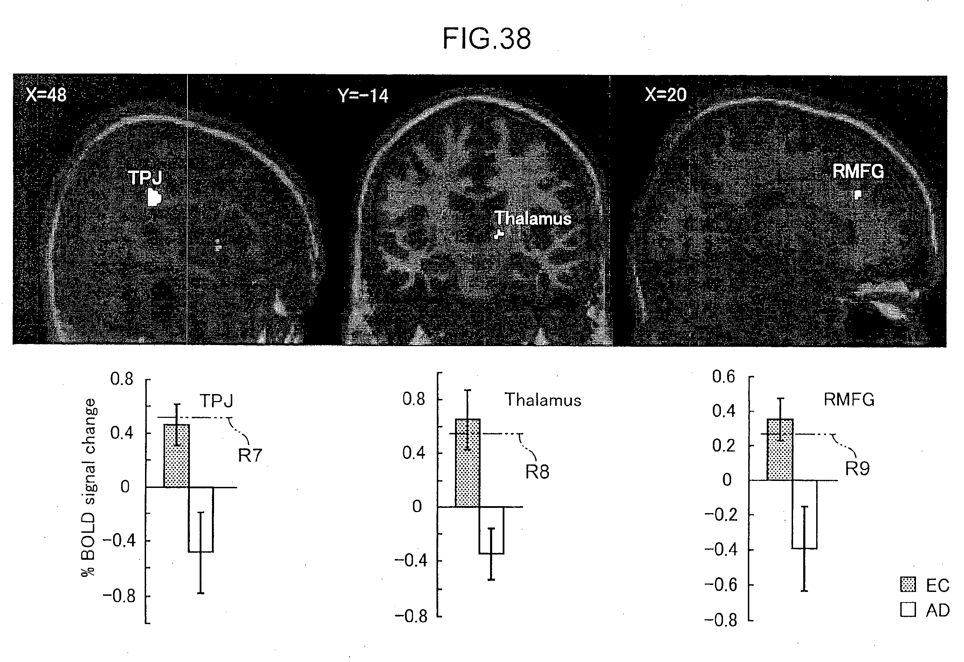

The storage unit 8 stores reference analysis values shown in Fig. 38 to Fig. 42 for comparison with the BOLD signal change rate computed by the active state analysis unit 40 (preparation step). Specifically, the storage unit 8 previously stores reference analysis values being previously computed BOLD signal change rates for a plurality of subjects H (subjects shown in Fig. 32 in this embodiment) belonging to two groups including AD and EC.

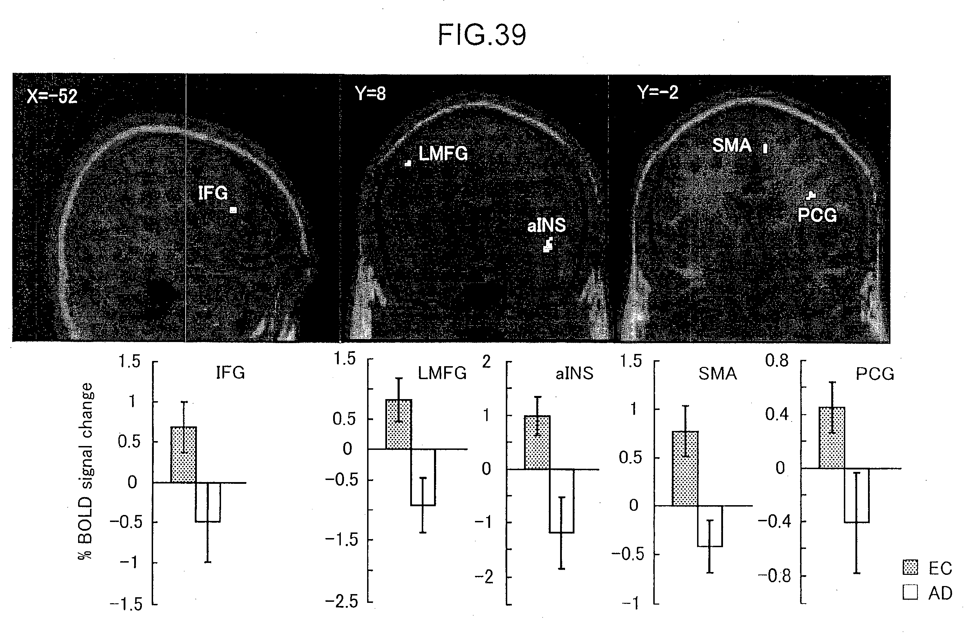

Fig. 38 and Fig. 39 each show activation sites of the brain and the BOLD signal change rate at a cue-target interval of 200 ms. The BOLD signal change rates of EC in the activation sites shown in Fig. 38 and Fig. 39 all change to a plus, and the BOLD signal change rates of AD in the activation sites all change to a minus. Significant differences exist between the BOLD signal change rates of EC and the BOLD signal change rates of AD at P value < 0.01. The activation sites shown in Fig. 38 are a TPJ, a Thalamus and a RMFG (white portions on colored backgrounds), and the activation sites shown in Fig. 39 are an IFG, a LMFG, an aINS, a SMA, and a PCG (white portions on colored backgrounds). Formal names of the activation sites will be listed later.

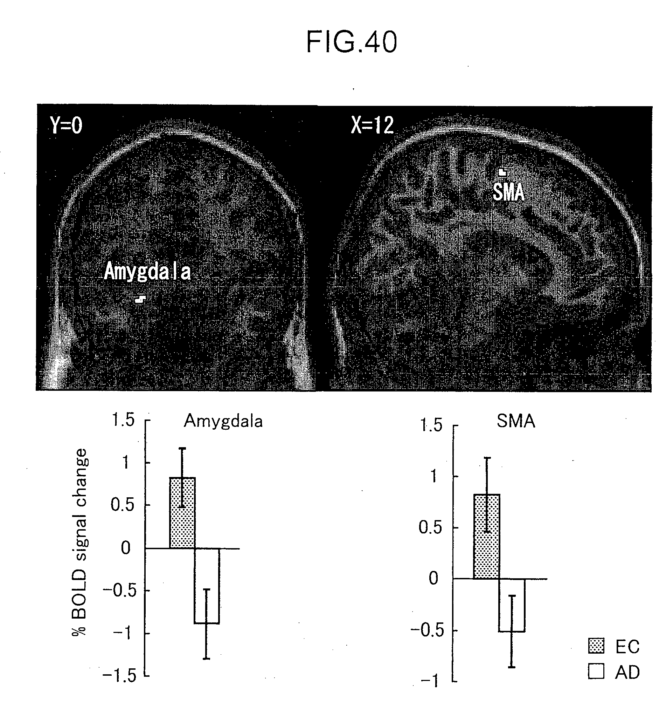

Fig. 40 is a figure showing activation sites of the brain and the BOLD signal change rate at a cue-target interval of 400 ms. The BOLD signal change rate of EC in the activation sites shown in Fig. 40 all change to a plus, and the BOLD signal change rates of AD in the activation sites all change to a minus. Significant differences exist between the BOLD signal change rates of EC and the BOLD signal change rates of AD at P value < 0.01. The activation sites shown in Fig. 40 are an Amygdala, and a SMA.

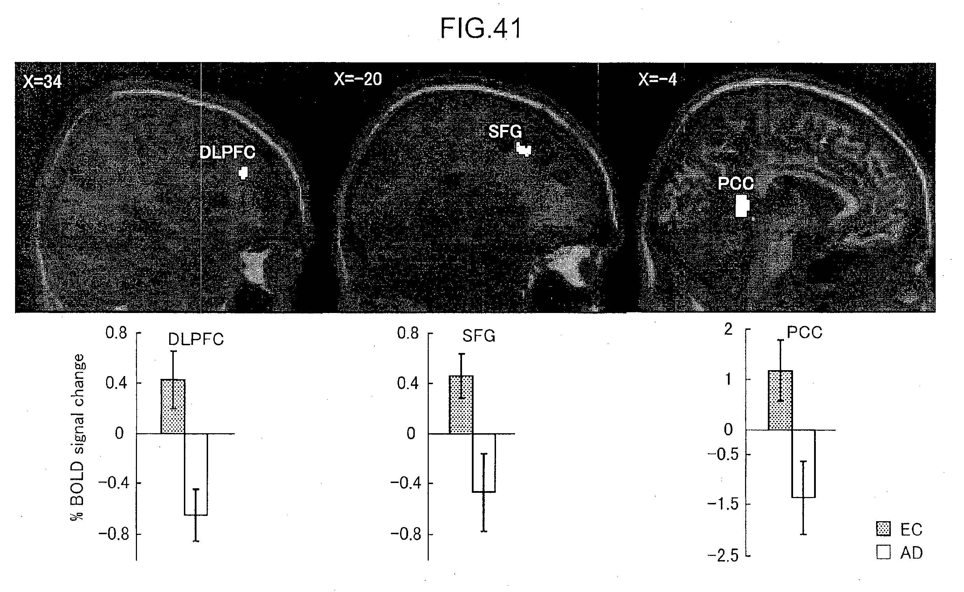

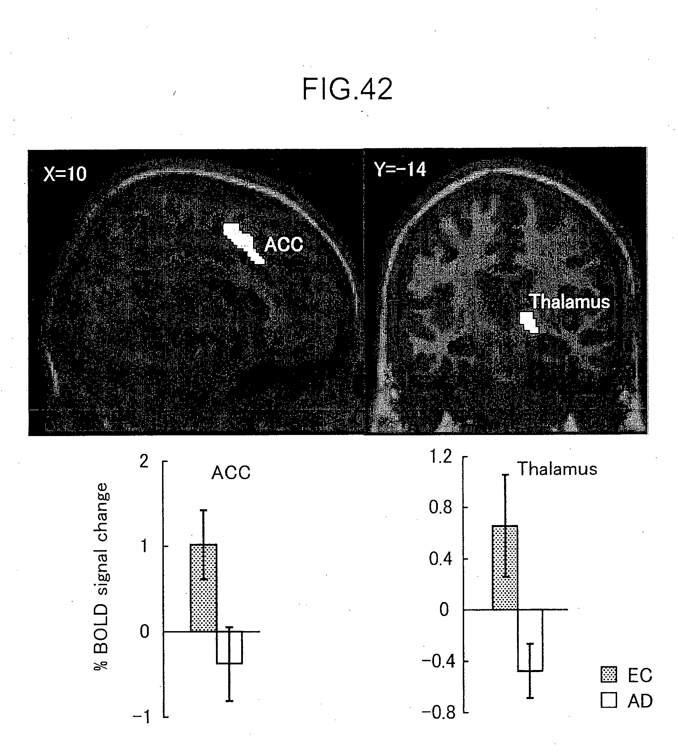

Fig. 41 and Fig. 42 each show activation sites of the brain and the BOLD signal change rate at a cue-target interval of 800 ms. The BOLD signal change rate of EC in the activation sites shown in Figs. 41 and Fig. 42 all change to a plus, and the BOLD signal change rates of AD in the activation sites all change to a minus. Significant differences exist between the BOLD signal change rates of EC and the BOLD signal change rates of AD at P value < 0.01. The activation sites shown in Fig. 41 and Fig. 42 are a DLPFC, a SFG, a PCC, an ACC, a Thalamus.

Formal names of activation sites

ACC: anterior cingulate cortex
aINS: anterior insula
Amygdala
DLPFC: dorsalateral prefrontal cortex
IFG: inferior frontal gyrus
RMFG: right middle frontal gyrus
LMFG: left middle frontal gyrus
PCC: posterior cingulated cortex

PCG: postcentral gyrus
SFG: superior frontal gyrus
SMA: supplementary motor area
Thalamus
TPJ: temporal parietal junction

The data creation unit 17 (see Fig. 37) creates data in which a BOLD signal change rate computed for the specified subject H is compared with the reference analysis values (Fig. 38 to Fig. 42) stored in the storage unit 8. Specifically, the data creation unit 17 creates a graph in which BOLD signal change rates R7 to R9 computed for the specified subject H are shown on a graph of the reference analysis values, and displays this graph as shown in Fig. 38. This graph may be displayed by the projector 32, but can also be displayed on the display 2, similarly to the embodiment.

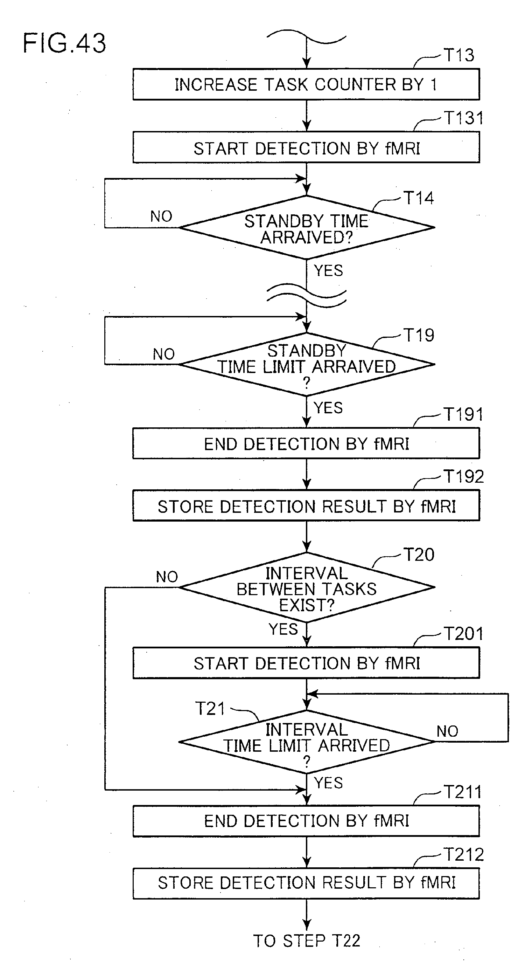

Hereinafter, processes performed by the control unit 35 will be described with reference to Fig. 43 to Fig. 44.

Main routine performed by the control unit 35 is similar to that shown in Fig. 19. Among information collecting processing T and information analysis processing U performed by the control unit 35, difference between Fig. 33 and Fig. 36 will be mainly described.

With reference to Fig. 43, after Step T13 of information collecting processing T is performed, detection by the fMRI 33 starts (Step T131). That is, when the tasks shown in Fig. 28 start, the detection of the fMRI 33 starts at the same time.

A process similar to that of Fig. 33 is thereafter performed. When it is determined that a standby time limit has arrived in Step T19, the detection by the fMRI 33 is terminated (Step T191), the detection result by the fMRI 33 is stored in the storage unit 8 (Step T192). Consequently, the detection by the fMRI 33 is performed during the execution period between the display of the cue images D61 and D62 and the reception of the response (first detection step).

Next, in a case where the interval between tasks exists (YES in Step T20), the detection by the fMRI 33 starts. When the interval is terminated (YES in Step T21), the detection by the fMRI 33 is terminated (Step T211), the detection result is stored in the storage unit 8 (Step T212). Consequently, it is possible to perform the detection by the fMRI 33 at the interval between the tasks (second detection step).

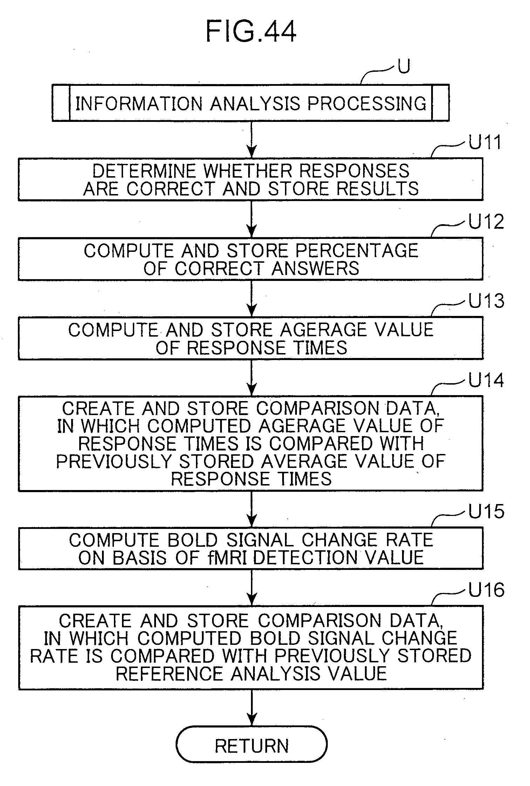

When the information collecting processing T is terminated, the information analysis processing shown in Fig. 44 is performed. Among the processes shown in Fig. 44, Steps U11 to U14 are similar to the processes shown in Fig. 35. Therefore, processes subsequent to Step U15 will be described as follows.

After Step U14 is performed, the BOLD signal change rate is computed on the basis of the detection value by the fMRI 33 (Step U15: active state analysis step). Then, comparison data, in which the computed BOLD signal change rate is compared with the reference analysis value, is created, and stored in the storage unit 8 (Step U16: comparison step). Specifically, in Step U16, as shown in Fig. 38, comparison data, in which the computed BOLD signal change rates R7 to R9 are superimposed on a graph of the reference average values, is created. Therefore, a doctor seeing this comparison data can determine the onset of dementia for the subject H.

As described above, in the embodiment, the BOLD signal change rates are computed for the specified subject H, and the BOLD signal change rates are compared with the reference analysis values. Consequently, it is possible to determine a dementia patient and people other than the dementia patient on the basis of the change in the active state of the brain.

The verification device 31 displays data (Fig. 38) in which the values measured for the specified subject H are compared with the values stored in the storage unit 8, but is not limited to this. The verification device 31 may determine which group (AD or EC) the specified subject H belongs to, and the determination result may be displayed.

Specifically, a threshold value for differentiating AD from EC can be previously prepared on the basis of the BOLD signal change rate (Fig. 38 to Fig. 42) stored in the storage unit 8, and be stored in the storage unit 8. Then, the data creation unit 17 can determine whether the specified subject H is AD or EC by comparing the BOLD signal change rate computed for the specified subject H with the threshold value, and display the determination result.

The above-mentioned specific embodiments mainly include the inventions having the following configurations.

The present invention provides a method for verifying onset of dementia of a subject, this method including: a stimulus provision step of providing the subject with one of two alternatively selectable selection items as a stimulus including at least one of audio information and visual information, a plurality of times; a measurement step of receiving a response from the subject with respect to the stimulus provided in the stimulus provision step, and measuring a response time from the provision of the stimulus to the reception of the response; an average value computation step of computing an average value of the response time measured in the measurement step; a preparation step of previously preparing reference average values with respect to a plurality of subjects including a dementia patient and a person other than the dementia patient, the reference average values being average values of the response times to the stimulus provided in the stimulus provision step; and a comparison step of comparing the average value of the response times obtained with respect to a specified subject in the average value computation step with the reference average values.

Additionally, the present invention provides a device for verifying onset of dementia for a subject, which includes: an information provision unit capable of providing the subject with one of two alternatively selectable selection items as a stimulus including at least one of audio information and visual information; an operation unit capable of outputting, in accordance with operation from the subject, a command indicating that one of the two selection items is selected; and a control unit controlling the information provision unit in order to provide the subject with the selection item, and analyzes information for verifying the onset of dementia on the basis of the command input from the operation unit, wherein the control unit has: a stimulus provision unit which provides a stimulus showing one of the two selection items through the information provision unit a plurality of times; a measurement unit which measures a response time from the provision of the stimulus by the stimulus provision unit to the reception of the command output from the operation unit; an average time computation unit which computes an average value of the response times measured by the measurement unit; a storage unit which previously stores reference average values previously prepared with respect to a plurality of subjects including a dementia patient and a person other than the dementia patient, the reference average values being average values of the response times to the stimulus provided by the stimulus provision unit; and a data creation unit which creates data, in which the average value of the response times obtained with respect to a specified subject by the average time computation unit is compared with the reference average values.

According to these inventions, it is possible to verify the onset of dementia of the specified subject by comparison between the reference average values of the response times previously prepared with respect to the plurality of subjects including the dementia patient and average values of response times obtained with respect to the specific subject.

Specifically, the inventors of this application have confirmed that in a case where the stimulus including at least one of the audio information and the visual information is supplied to the subject a plurality of times, significant differences in the response time exist between Alzheimer's disease (hereinafter, often referred to as AD) patients, and people other than the AD patients (see Fig. 8, Fig. 9 and Fig. 31). Therefore, as in the present invention, it is possible to verify the onset of dementia by comparing the previously prepared reference average values with the average value computed with respect to the specified subject.

Particularly, in the present invention, the subject is required to select one selection (response) of the two alternatively selectable selection items. Therefore, the plurality of subjects can respond at relatively even speed regardless of interest or concern to the provided information, unlike a conventional case where a meaningful object is specified.

Accordingly, according to the present invention, information (response times) for verifying the onset of dementia can be obtained while suppressing individual differences between the subjects.

In the present invention, the 'Alzheimer's disease' is a symptom satisfying all of the following A to F according to the DSM-IV (The Diagnostic and Statistical Manual of Mental Disorders-IV).

A. At least any one of symptoms of memory impairment, aphasia, apraxia, agnosia, and behavioral dysfunction is revealed by onset of varied cognitive losses.

B. The cognitive losses of A cause significant impaired social function.

C. Progression is characterized by gradual onset and continuous reduction of cognition.

D. The cognitive losses of A are not caused by other dementia.

E. The cognitive losses of A are not symptoms which appear only during the course of delirium.

F. The disease by this dementia cannot be explained well by other axis I diseases (schizophrenia, etc.).

Additionally, in the present invention, the 'two alternatively selectable selection items' means, for example, character, figures, symbols or combination thereof, meaning opposing directions such as right and left, and top and bottom or opposing contents such as ox and ON/OFF.

In the method for verifying onset of dementia, in the stimulus provision step, a target stimulus which provides one of the two selection items as the visual information is preferably provided a plurality of times, and a cue stimulus which is the visual information instructing one of the two selection items is preferably provided for a preset period before providing the target stimuli for each of the plurality of times, and in the measurement step, a time from the provision of the target stimulus to the reception of the response is preferably measured as the response time.

Additionally, in the device for verifying onset of dementia, the information provision unit preferably has a visual information provision unit capable of providing the subject with the visual information, the stimulus provision unit preferably provides a target stimulus which provides one of the two selection items as the visual information a plurality of times, and provides a cue stimulus which is the visual information instructing one of the two selection items for a preset period before providing the target stimuli for each of the plurality of times, and the measurement unit preferably measures a time from the provision of the target stimulus to the reception of the command output from the operation unit as the response time.

According to these aspects, the target stimulus is provided after providing the cue stimulus, and hence more valuable information for verifying the onset of dementia can be collected.

Specifically, the inventors of this application have confirmed that under a situation where the cue stimulus is provided as a cue of the item indicated by the target stimulus, significant differences in the average value of the response times to the target stimulus exist between the dementia patients and the people other than the dementia patients (see Fig. 31). It is considered that the reason why the significant difference thus exists is "attention" paid when a human visually collects information from outside. Specifically, the "attention" includes passive attention which is paid when reflexively turning attention to sudden change of an uncontemplated stimulus, and active attention which is paid when consciously turning attention to a certain position. Here, in each of the respective aspects, the active attention of the subject is paid by instructing one of two alternatively selectable selection items by the cue stimulus. It is considered that people other than the dementia patients have higher ability for introducing an item indicated by the target stimulus with the active attention paid than dementia patients, and hence the significant difference exists.

Accordingly, the onset of dementia can be more reliably verified by comparing the average value of the response times measured for the specified subject with the reference average values.

Particularly, according to the aspects, the target stimulus and the cue stimulus are each provided as the visual information. Accordingly, test contents can be further simplified compared to a case of providing the subject with the audio stimulus in addition to the visual stimulus.

In the method for verifying onset of dementia, the plurality of times of the cue stimuli provided in the stimulus provision step preferably include a validity cue stimulus which previously instructs an item indicated by the target stimulus, and an invalidity cue stimulus which previously instructs an item opposite to the item indicated by the target stimulus, in the average value computation step, an average value of the response times excluding the response time to the target stimulus after the provision of the invalidity cue stimulus is preferably computed, and in the preparation step, a reference average value excluding the response time to the target stimulus after the provision of the invalidity cue stimulus is preferably computed.

Additionally, in the device for verifying onset of dementia, the cue stimuli preferably include a validity cue stimulus which previously instructs an item indicated by the target stimulus, and an invalidity cue stimulus which previously instructs an item opposite to the item indicated by the target stimulus, the average time computation unit preferably computes an average value of the response times excluding the response time to the target stimulus after the provision of the invalidity cue stimulus, and the storage unit preferably previously stores a reference average value excluding the response time to the target stimulus after the provision of the invalidity cue stimulus.

According to these aspects, the cue stimuli include the validity cue stimulus and the invalidity cue stimulus, and the reference average values and the average value excluding the response time to the target stimulus after the provision of the invalidity cue stimulus. Thus, the validity cue stimulus and the invalidity cue stimulus are mixed, thereby enabling the subject to be inhibited from responding on the basis of only the cue stimulus without paying attention to the target stimulus. Additionally, the response time corresponding to ability for introducing the item indicated by the target image with the active attention paid can be reliably obtained by excluding the response times to the target stimulus after the provision of the invalidity cue stimulus from an object to be compared.

In the method for verifying onset of dementia, in the stimulus provision step, a preset interval is preferably inserted between the cue stimulus and the target stimulus.

Additionally, in the device for verifying onset of dementia, the stimulus provision unit preferably provides the target stimulus at a preset interval after the provision of the cue stimulus.

According to these aspects, the interval is provided between the cue stimulus and the target stimulus. Consequently, the standby time for target stimulus can be given to the subject with the active attention paid by the cue stimulus, unlikely to a case of continuously providing the cue stimulus and the target stimulus. Accordingly, it is possible to suppress variation in the response times by confusion of the cue stimulus and the target stimulus.

The method for verifying onset of dementia preferably further includes a correct answer percentage computation step of computing a percentage of correct answers for the responses from the subject, wherein in the preparation step, the reference average value is preferably prepared with respect to the subject whose percentage of correct answers is equal to or more than 70% among a plurality of the subjects.

Additionally, the device for verifying onset of dementia preferably further includes a correct answer percentage computation unit which computes a percentage of correct answers for the responses from the subject, wherein the storage unit preferably previously stores the reference average value with respect to the subject whose percentage of correct answers is equal to or more than 70% among a plurality of the subjects.

According to these aspects, the response times by the subjects whose percentage of correct answers is equal to or more than 70% are used as the reference average value. Consequently, the reference average value more accurately indicates the response time to the target stimulus with the active attention paid. Specifically, the target stimuli indicate the two alternatively selectable selection items, and hence the percentage of correct answers is 50% even when the response is made without verifying the target stimulus. Therefore, as in the aspects, the response times, for which the percentage of correct answers is 70% or more, are extracted, thereby enabling computation of the reference average value based on the response times in which the subject is likely to respond while verifying the target stimulus.

The method for verifying onset of dementia preferably further includes: a first detection step of detecting oxygen consumption and blood flow of a brain of the subject by using an fMRI during an execution period in which the stimulus provision step and the measurement step are executed; a second detection step of detecting the oxygen consumption and the blood flow of the brain of the subject by using the fMRI during a period other than the execution period; and an active state analysis step of analyzing a change in an active state of the brain of the subject between the execution period and the period other than the execution period on the basis of detection values in the first detection step and the second detection step, wherein in the preparation step, reference analysis values, obtained by analysis of the change in the active state of the brain with respect to a plurality of subjects including a dementia patient and a person other than the dementia patient, are preferably previously prepared, and in the comparison step, the change in the active state of the brain obtained with respect to the specified subject in the active state analysis step is preferably compared with the reference analysis values.

Additionally, the device for verifying onset of dementia preferably further includes an fMRI which detects oxygen consumption and blood flow of a brain of the subject; and an active state analysis unit which analyzes a change in an active state of the brain of the subject on the basis of a detection value by the fMRI during an execution period from the provision of the cue stimulus by the stimulus provision unit to the reception of the command output from the operation unit, and a detection value by the fMRI during a period other than the execution period, wherein the storage unit preferably previously stores reference analysis values obtained by analysis of the change in the active state of the brain with respect to a plurality of subjects including a dementia patient and a person other than the dementia patient, and the data creation unit preferably creates data, in which the change in the active state of the brain obtained with respect to the specified subject by the active state analysis unit is compared with the reference analysis values.

According to these aspects, the dementia patients and people other than the dementia patients can be determined on the basis of the change in the active state of the brain. The reason will be as follows.

The inventors of this application have confirmed that the change in the active state of the brain between the execution period from the provision of the cue stimulus to the reception of the response, and the period other than the execution period is different between the dementia patients and the people other than the dementia patients (see Fig. 38 to Fig. 42). Specifically, when the stimulus is applied to the brain (at the time of activation), the amount of deoxyhemoglobin (hereinafter referred to as Deoxy-Hb) in the brain increases with increase in oxygen consumption of the brain, and the amount of oxyhemoglobin (hereinafter referred to as Oxy-Hb) increases with increase in blood flow of the brain. More specifically, the increased amound of Oxy-Hb being a diamagnetic substance is extremely large relative to the increase in Deoxy-Hb being a paramagnetic substance. Consequently, so-called BOLD (Blood Oxygenation Level Dependent) effect involving enhancement of the MRI signals and extension of relaxation time of MRI signals is produced. This BOLD effect is detected by using the fMRI (functional Magnetic Resonance Imaging), the change in the active state of the brain can be analyzed. Then, the change of this BOLD effect shows different behavior between the dementia patients and the people other than the dementia patients in the specified activation sites of the brain (see Fig. 38 to Fig. 42). Accordingly, the onset of dementia can be verified by comparing the previously prepared (stored) reference analysis values with the change of the active state of the brain obtained with respect to the specified subject.

The method for verifying onset of dementia preferably further includes a selection step of selecting one of three kinds of conditions including an audio selective attention condition in which attention is paid only to information obtained through an auditory sense to respond, a visual selective attention condition in which attention is paid only to information obtained through a visual sense to respond, and an audio-visual division attention condition in which attention is paid only to information obtained through the auditory sense and the visual sense to respond, and an offer step of offering the condition selected in the selection step to the subject, wherein in the stimulus provision step, three kinds of stimuli including an audio stimulus which provides one of the two selection items as the audio information, a visual stimulus which provides one of the two selection items as the visual information, and an audio-visual stimulus which provides one of the two selection items as the audio information and the visual information are preferably provided to the subject, a plurality of times, at preset respective response periods under the condition selected in the selection step, in the measurement step, the response time to at least one kind of stimulus among the three kinds of stimuli provided in the stimulus provision step is preferably measured, in the preparation step, average values of the response times to the respective stimuli in each of the three kinds of conditions are preferably previously prepared as the reference average value, and in the comparison step, the average value of the response times obtained with respect to the specified subject in the average value computation step is compared with the average value of the response times to the same stimulus in the same condition among the reference average values.

Additionally, in the device for verifying onset of dementia, an information provision unit preferably includes an audio information provision unit capable of providing a subject with audio information, and a visual information provision unit capable of providing the subject with visual information, the control unit preferably further includes a test condition selection unit which selects one of three kinds of conditions including an audio selective attention condition in which attention is paid only to information obtained through an auditory sense to respond, a visual selective attention condition in which attention is paid only to information obtained through a visual sense to respond, and an audio-visual division attention condition in which attention is paid only to information obtained through the auditory sense and the visual sense to respond, and offers the selected condition to the subject through at least one of the audio information provision unit and the visual information provision unit, the stimulus provision unit preferably provides, a plurality of times, the subject with three kinds of stimuli including an audio stimulus which provides one of the two selection items through the audio information provision unit, a visual stimulus which provides one of the two selection items through the visual information provision unit, and an audio-visual stimulus which provides one of the two selection items through the audio information provision unit and the visual information provision unit, the measurement unit is preferably a measurement unit which measures the response time to at least one kind of stimulus among the three kinds of stimuli provided from the stimulus provision units, the storage unit previously preferably stores average values of the response times to the respective stimuli in each of the three kinds of conditions as the reference average value, and the data creation unit preferably creates data, in which the average value of the response times obtained with respect to the specified subject by the average time computation unit is compared with the average value of the response times to the same stimulus in the same condition among the reference average values.

According to these aspects, the onset of dementia can be verified by using the audio stimulus, the visual stimulus, and the audio-visual stimulus.

Specifically, the inventors of this application have confirmed that the significant differences in the response time exist between the dementia patients and the people other than the dementia patients by supplying the three stimuli , a plurality of times, to the subjects under one condition selected from the three conditions (see Fig. 8 and Fig. 9). Therefore, as in the aspects, it is possible to verify the onset of dementia by comparing the previously prepared reference average values with the average value computed with respect to the specified subject.

In the respective aspects, the 'at least one kind of the stimulus among the three kinds of stimuli' means at least one of the audio stimulus and the audio-visual stimulus in the audio selective attention condition, at least one of the visual stimulus and the audio-visual stimulus in the visual selective attention condition, and at least one of the audio stimulus, the visual stimulus, and the audio-visual stimulus in the audio-visual division attention condition.

Additionally, in the respective aspects, the 'response times to the respective stimuli in each of the three kinds of conditions' means the response times to the audio stimulus and the audio-visual stimulus in the audio selective attention condition, the response times to the visual stimulus and the audio-visual stimulus in the visual selective attention condition, and the response times to the audio stimulus, the visual stimulus, and the audio-visual stimulus in the audio-visual division attention condition. That is, it is assumed that the response to the visual stimulus in the audio selective attention condition is 'no response', for example, and therefore the response to the visual stimulus in this case is excluded. The response to the visual stimulus in the audio selective attention condition is similar to this.

In the method for verifying onset of dementia, in the selection step, after performing the measurement step in a condition selected last time, a condition other than the condition selected last time is preferably newly selected among the three kinds of conditions, and the offer step, the stimulus provision step, and the measurement step are preferably performed with respect to the condition newly selected in the selection step.

Additionally, in the device for verifying onset of dementia, the test condition selection unit preferably newly selects a condition other than a condition selected last time among the three kinds of conditions, after completion of the measurement of the response time in the condition selected last time by the measurement unit.

According to these aspects, the average values of the response times under 2 or more conditions can be obtained. Therefore, it is possible to more accurately verify the onset of dementia by increasing the number of the average values compared with the reference average values.

In the method for verifying onset of dementia, the response times to respective stimuli in the each of respective three kinds of conditions are preferably measured with respect to the specified subject by performing, in three cycles, the selection step, the offer step, the stimulus provision step, and the measurement step.

In the device for verifying onset of dementia, the test condition selection unit preferably sequentially selects all of the three kinds of conditions with respect to the specified subject, and the measurement unit preferably measures response times to the respective stimuli in each of the three kinds of conditions.

Thus, it is possible to obtain the average value of the average values with respect to all combination of the three kinds of conditions and the three kinds of stimuli.

In the method for verifying onset of dementia, in the selection step, at least the audio selective attention condition and the visual selective attention condition are preferably sequentially selected, and in the measurement step, the respective response times of the subject to the audio stimulus and the audio-visual stimulus are preferably measured in the audio selective attention condition, and the respective response times of the subject to the visual stimulus and the audio-visual stimulus are measured in the visual selective attention condition, the method preferably further includes a first index computation step of computing a promoting effect index indicating a degree of a promoting effect by audio-visual integration on the basis of the response times measured in the measurement step, wherein in the preparation step, the promoting effect index is preferably prepared in advance with respect to the plurality of subjects including a mild cognitive impairment patient and a healthy elderly person, and in the comparison step, the promoting effect index computed in the first index computation step is preferably compared with the promoting effect index prepared in the preparation step.

Additionally, in the device for verifying onset of dementia, the test condition selection unit preferably sequentially selects at least the audio selective attention condition and the visual selective attention condition, the measurement unit preferably measures the respective response times to the audio stimulus and the audio-visual stimulus in the audio selective attention condition, and measures the respective response times to the visual stimulus and the audio-visual stimulus in the visual selective attention condition, the control unit preferably further includes a first index computation unit which computes a promoting effect index indicating a degree of a promoting effect by audio-visual integration on the basis of the response times measured by the measurement unit, the storage unit preferably previously stores the promoting effect index with respect to the plurality of subjects including a mild cognitive impairment patient and a healthy elderly person, and the data creation unit preferably creates data, in which the promoting effect index computed by the first index computation unit is compared with the promoting effect index stored in the storage unit.

In these aspects, the previously prepared promoting effect index indicating the degree of the promoting effect by audio-visual integration is compared with the promoting effect index indicating the degree of the promoting effect by audio-visual integration, computed with respect to the specified subject. Consequently, it is possible to distinguish between the mild cognitive impairment (hereinafter, referred to as MCI) patients, and the healthy elderly people (Elderly).

Specifically, the inventors of this application faced a problem that in a case of performing the comparison of the response times described above, while it is possible to distinguish between the AD patients and the people other than the AD patients, it is difficult to sufficiently distinguish between the MCI patients and the healthy elderly people (see an E-M column of the audio-visual attention condition of Fig. 9). Therefore, the inventors of this application have conceived of a method of comparing promoting effect indexes indicating the degree of the promoting effect by the audio-visual integration, by focusing on the following (1) to (4).

(1) A speed of the response time in a case where the audio-visual stimulus complying with a theory of meaning is provided is faster than a speed of the response time in a case where the visual stimulus and the audio stimulus are separately provided (Jennifer L. Mozolic, Christina E. Hugenschmidt, Ann M. Peiffer, Paul J. Laurienti,: Modality-specific selective attention attenuates audiovisual integration, Experimental Brain Research, (2008) 184: 39 - 52).

(2) The promoting effects in elderly people are higher than the promoting effects in young people (Paul J. Laurienti, Jonathan H. Burdette, Joseph A. Maldjian, Mark T. Wallace,: Enhanced audiovisual integration in older adults, Neurobiology of Aging (2006) 27: 1155 - 1163).

(3) As to cerebral metabolic rates (rCMRglc) of glucose of brain sites for the audio-visual stimulus, the rCMRglc of the dementia patients is lower than that of the healthy people of the same age. Furthermore, it is found that there is a correlation between the degree of severity of dementia and a lowering rate of the rCMRglc (Pietro Pietrini, Gene E. Alexander, Maura L. Furey, Alessio Darn, Marc J. Mentis, Barry Horwitz, Mario Guazzehhi, Mark B. Schapiro, and Stanley I. Rapoport,: Cerebral Metabolic Response to Passive Audiovisual Stimulation in Patients with Alzheimer's Disease and Healthy Volunteers Assessed by PET, THE JOURNAL OF NUCLEAR MEDICINE, (2000) 41: 575 - 583).

(4) The ability of the audio-visual integration of the schizophrenia patients are lower than that of the healthy people (J.J. de Jong, P.P.G. Hodiamont J. Van den Stock, B. de Gelder, Audiovisual emotion recognition in schizophrenia: Reduced integration of facial and vocal affect, SCHIZOPHRENIA RESEARCH, (2009) 107: 286 - 293).

That is, according to the respective aspects, it is possible to distinguish between the healthy elderly people and the MCI patients by comparison of the promoting effects having a property of showing an higher effect with increase in person ages, and reducing with reduction in brain function.

The 'audio-visual integration' means that information to an auditory sense and information to a visual sense interact.

Additionally, the 'promoting effect' means that pieces of information obtained from 2 or more intervals enhance each other. Specifically, the 'promoting effect' is observed in a case where audio information and visual information complying with a theory of meaning are provided, like a case where a red visual stimulus and an audio stimulus of a word "red" are provided at the same time. It is known that the promoting effect varies depending on the attention condition, and is higher under the audio-visual division attention condition compared to a case where the attention to the auditory sense or the visual sense is selectively paid, in a case where the visual information and the audio information are provided (see the exhibition (3)).

Furthermore, the 'mild cognitive impairment' means that while ability such as cognitive functions and memory declines compared to ability expected in the course of normal aging, the symptom is less severe than dementia. Additionally, it is known that the mild cognitive impairment patients have higher possibility of the onset of dementia than the healthy elderly people. According to operational diagnosis criteria of amnestic mild cognitive impairment proposed in MCI consensus conference (Chicago, 1999), a person is diagnosed with MCI in a case of falling under the following A to E.
A. Individuals and families complain of a memory loss.
B. A general cognitive function is normal.
C. Activities of daily life are independent.
D. There is no possibility of dementia.
E. Memory impairment, which cannot be explained only by influences of age or an education level, exists.

Additionally, the 'healthy elderly people' means people with no physical and mental imperfections, particularly with no abnormalities of neuronal functions of a visual sense, an auditory sense, memory and the like.
In the method for verifying onset of dementia, an audio cumulative distribution probability of the response times to the audio stimulus in the audio selective attention condition, a visual cumulative distribution probability of the response times to the visual stimulus in the visual selective attention condition, a first audio-visual cumulative distribution probability of the response times to the audio-visual stimulus in the audio selective attention condition, a second audio-visual cumulative distribution probability of the response times to the audio-visual stimulus in the visual selective attention condition, an estimate value of the cumulative distribution probability of the response times to the audio-visual stimulus on the basis of the audio cumulative distribution probability and the visual cumulative distribution probability, a first subtraction value as the promoting effect index obtained by subtracting the estimate value from the first audio-visual cumulative distribution probability, and a second subtraction value as the promoting effect index obtained by subtracting the estimate value from the second audio-visual cumulative distribution probability are preferably computed in the first index computation step.
Additionally, in the device for verifying onset of dementia, a first index computation unit preferably computes an audio cumulative distribution probability of the response times to the audio stimulus in the audio selective attention condition, a visual cumulative distribution probability of the response times to the visual stimulus in the visual selective attention condition, a first audio-visual cumulative distribution probability of the response times to the audio-visual stimulus in the audio selective attention condition, a second audio-visual cumulative distribution probability of the response times to the audio-visual stimulus in the visual selective attention condition, an estimate value of the cumulative distribution probability of the response times to the audio-visual stimulus on the basis of the audio cumulative distribution probability and the visual cumulative distribution probability, a first subtraction value as the promoting effect index obtained by subtracting the estimate value from the first audio-visual cumulative distribution probability, and a second subtraction value as the promoting effect index obtained by subtracting the estimate value from the second audio-visual cumulative distribution probability.

According to these aspects, the first subtraction value and the second subtraction value being the indexes of the promoting effects can be obtained on the basis of the estimate value of the cumulative distribution probability of the response times to the audio-visual stimulus, and the actual measured value of the cumulative distribution probability of the response times to the audio-visual stimulus (audio-visual cumulative distribution probability).

The 'cumulative distribution probability' is a rate of the sum total of data of all measured response times, which is sorted into each of segments and exists from the start of the stimulus provision to the segment, to the total number. The segment is configured by dividing a period between the start of the stimulus provision and the end of the preset response period into a plurality of equal response periods.

Additionally, the 'estimate value' can be computed, for example, by using a race model (COGNITIVE PSYCHOLOGY 14, 247-279 (1982): JEFF MILLER). Here, the race model is a model for computing the estimate value of the audio-visual cumulative distribution probability being a logical sum of the cumulative distribution probabilities on the basis of the audio cumulative distribution probability and the visual cumulative distribution probability.

In the method for verifying onset of dementia, in the selection step, the audio-visual division attention condition is preferably selected, and in the measurement step, the response times to the respective three kinds of stimuli are preferably measured, and the meehod preferably further includes a second index computation step of computing a promoting effect index indicating a degree of a promoting effect by audio-visual integration on the basis of the response times measured in the measurement step, in the preparation step, the promoting effect index is preferably prepared in advance with respect to the plurality of subjects including a mild cognitive impairment patient and a healthy elderly person, and in the comparison step, the promoting effect index computed in the second index computation step is preferably compared with the promoting effect index prepared with the preparation step.

Additionally, in the device for verifying onset of dementia, the test condition selection unit preferably selects the audio-visual division attention condition, the measurement unit preferably measures the response times to the respective three kinds of stimuli, the control unit preferably further includes a second index computation unit which computes a promoting effect index indicating a degree of a promoting effect by audio-visual integration on the basis of the response times measured in the measurement unit, the storage unit previously preferably stores the promoting effect index with respect to the plurality of subjects including a mild cognitive impairment patient and a healthy elderly person, the data creation unit preferably creates data, in which the promoting effect index computed by the second index computation unit is compared with the promoting effect index stored in the storage unit.

According to these aspects, it is possible to shorten the time for obtaining the promoting effect index. Specifically, in the respective aspects, as described above, it is possible to obtain the promoting effect index by measuring the response time only by the audio-visual division attention condition, unlikely a case where the response time is measured under two of the audio selective attention condition and the visual selective attention condition.

In the method for verifying onset of dementia, an audio cumulative distribution probability of the response times to the audio stimulus in the audio-visual division attention condition, a visual cumulative distribution probability of the response times to the visual stimulus in the audio-visual division attention condition, an audio-visual cumulative distribution probability of the response times to the audio-visual stimulus in the audio-visual division attention condition, an estimate value of the cumulative distribution probability of the response times to the audio-visual stimulus on the basis of the audio cumulative distribution probability and the visual cumulative distribution probability, and a subtraction value as the promoting effect index obtained by subtracting the estimate value from the audio-visual cumulative distribution probability are preferably computed in the second index computation step.

In the device for verifying onset of dementia, the second index computation unit preferably computes an audio cumulative distribution probability of the response times to the audio stimulus in the audio stimulus in the audio-visual division attention condition, a visual cumulative distribution probability of the response times to the visual stimulus in the audio-visual division attention condition, an audio-visual cumulative distribution probability of the response times to the audio-visual stimulus in the audio-visual division attention condition, an estimate value of the cumulative distribution probability of the response times to the audio-visual stimulus on the basis of the audio cumulative distribution probability and the visual cumulative distribution probability, and a subtraction value as the promoting effect index obtained by subtracting the estimate value from the audio-visual cumulative distribution probability.

According to these aspects, it is possible to obtain the subtraction value being the index of the promoting effect on the basis of the estimate value of the cumulative distribution probability of the response times to the audiovisual stimulus, and the actual measured value of the cumulative distribution probability of the response times to the audio-visual stimulus (audio-visual cumulative distribution probability).

The method for verifying onset of dementia preferably further includes a correct answer percentage computation step of computing a percentage of correct answers in the responses from the subject, wherein in the preparation step, reference percentages of correct answers in the responses from the plurality of subjects are preferably previously prepared, and in the comparison step, the percentage of correct answers obtained with respect to the specified subject in the correct answer percentage computation step is preferably compared with the percentage of correct answers to the same stimulus in the same condition among the reference percentages of correct answers.

In the device for verifying onset of dementia, the control unit preferably further includes a correct answer percentage computation unit which computes a percentage of correct answers with respect to the responses from the subject, the storage unit preferably stores reference percentages of correct answers prepared with respect to the responses from the plurality of subjects, and the comparison unit preferably compares the percentage of correct answers obtained with respect to the specified subject by the correct answer percentage computation unit with the percentage of correct answers to the same stimulus in the same condition among the reference percentages of correct answers stored in the storage unit.

According to these aspects, it is possible to verify the onset of dementia for the specified subject. Specifically, the inventors of this application have confirmed that the significant differences in the percentage of correct answers exist between the AD patients and the people other than the AD patients by supplying the three stimuli under one condition selected from the three conditions at the intervals of a preset response period a plurality of times (see Fig. 10 and Fig. 11). Therefore, as in the present invention, it is possible to verify the onset of AD by comparing the previously prepared reference percentage of correct answers with the percentage of correct answers computed with respect to the specified subject.

In the device for verifying onset of dementia, the visual information provision unit is preferably configured from an image display device capable of displaying a preset image, and the data creation unit preferably causes the image display device to display created data.

According to this aspect, the data, in which the measured average value of the response times is compared with the previously stored average value of the response times, is displayed on the image display unit. Therefore, a healthcare worker seeing this image display unit can determine the onset of dementia.

In the device for verifying onset of dementia, the visual information provision unit is preferably configured from an image display device capable of displaying a preset image, and the test condition selection unit preferably displays two images each showing an ear under the audio selective attention condition, displays two images each showing an eye under the visual selective attention condition, and displays one image showing the ear and one image showing the eye under the audio-visual division attention condition.

According to this aspect, display of two images each showing an ear enables the subject to recognize the audio selective attention condition. Additionally, display of two images each showing an eye enables the subject to recognize the visual selective attention condition. Furthermore, display of one image showing an ear and one image showing an eye enables the subject to recognize the audio-visual division attention condition.

In the device for verifying onset of dementia, the storage unit preferably previously stores a threshold value for differentiating the average value of the response times previously computed with respect to the dementia patient, and the average value of the response times previously computed with respect to the person other than the dementia patient, and the data creation unit preferably determines whether or not the specified subject is a dementia patient, by comparing the average value of the response times computed with respect to the specified subject with the threshold value.

According to this aspect, it is possible to determine whether or not the specified subject is a dementia patient, by measuring the response time with respect to the specified subject.

In the device for verifying onset of dementia, the storage unit preferably previously stores a threshold value for differentiating the promoting effect index previously computed with respect to a healthy elderly person from the promoting effect index previously computed with respect to a mild cognitive impairment person, and the data creation unit preferably determines whether the specified subject is a healthy elderly person or a mild cognitive impairment person, by comparing the promoting effect index computed with respect to the specified subject with the threshold value.

According to this aspect, it is possible to determine whether the specified subject is a healthy elderly person or the mild cognitive impairment patient, by computing the promoting effect index with respect to the specified subject.

Industrial Applicability

According to the present invention, it is possible to obtain information for verifying onset of dementia while suppressing individual differences between subjects.

Explanation of Reference Numerals

D1 to D3 CONDITION IMAGE
D4 STIMULUS IMAGE
D5 BACKGROUND IMAGE
D61, D62 CUE IMAGE (CUE STIMULUS)
D71, D72 TARGET IMAGE (TARGET STIMULUS)
G1, G8 AUDIO CUMULATIVE DISTRIBUTION PROBABILITY
G2, G9 VISUAL CUMULATIVE DISTRIBUTION PROBABILITY
G3, G4, G10 CUMULATIVE DISTRIBUTION PROBABILITY
G5, G11 ESTIMATE VALUE
G6, G7, G12 SUBTRACTION VALUE
H SUBJECT
R1 AVERAGE VALUE
R2 PERCENTAGE OF CORRECT ANSWERS
R3 SUBTRACTION VALUE
1,21,31 VERIFICATION DEVICE
2 DISPLAY (VISUAL INFORMATION PROVISION UNIT, IMAGE DISPLAY DEVICE)
3 EAR PAD UNIT (AUDIO INFORMATION PROVISION UNIT)
4, 24 OPERATION UNIT
5,25,35 CONTROL UNIT
8 STORAGE UNIT
11 TEST CONDITION SELECTION UNIT
12 STIMULUS PROVISION UNIT
13 MEASUREMENT UNIT
14 AVERAGE TIME COMPUTATION UNIT
15 CORRECT ANSWER PERCENTAGE COMPUTATION UNIT
16 INDEX COMPUTATION UNIT
17 DATA CREATION UNIT
32 PROJECTOR (VISUAL INFORMATION PROVISION UNIT, IMAGE DISPLAY DEVICE)
37 INFORMATION ANALYSIS UNIT
40 ACTIVE STATE ANALYSIS UNIT

**Claims**

1. A device for verifying onset of dementia for a subject, the device comprising:

an information provision unit having a visual information provision unit capable of providing the subject with one of two alternatively selectable selection items as a stimulus including visual information;
an operation unit capable of outputting, in accordance with operation from the subject, a command indicating that one of the two selection items is selected; and
a control unit capable of controlling the information provision unit in order to provide the subject with the selection item, and capable of analyzing information for verifying the onset of dementia on the basis of the command input from the operation unit, wherein the control unit has:

a stimulus provision unit capable of providing a target stimulus providing one of the two selection items as visual information through the information provision unit a plurality of times, and the stimulus provision unit capable of providing a cue stimulus being visual information instructing one of the two selection items for a preset period through the information provision unit, before providing the target stimuli for each of the plurality of times;
a measurement unit capable of measuring a response time from the provision of the target stimulus by the stimulus provision unit to the reception of the command output from the operation unit;
an average time computation unit capable of computing an average value of the response times measured by the measurement unit;
a storage unit capable of previously storing reference average values previously prepared with respect to a plurality of subjects including a dementia patient and a person other than the dementia patient, the values being average values of the response times to the stimulus provided by the stimulus provision unit; and a data creation unit capable of creating data, in which the average value of the response times obtained with respect to a specified subject by the average time computation unit is compared with the reference average values.

2. The device for verifying onset of dementia according to claim 1, wherein the cue stimuli include a validity cue stimulus capable of previously instructing item indicated by the target stimulus, and an invalidity cue stimulus capable of previously instructing an item opposite to the item indicated by the target stimulus, the average time computation unit is capable of computing an average value of the response times excluding the response time to the target stimulus after the provision of the invalidity cue stimulus, and the storage unit is capable of previously storing a reference average value excluding the response time to the target stimulus after the provision of the invalidity cue stimulus.

## Patentansprüche

1. Vorrichtung für den Nachweis des Einsetzens von Demenz für ein Subjekt, wobei die Vorrichtung umfaßt:

eine Informationsbereitstellungseinheit, die eine visuelle Informationsbereitstellungseinheit aufweist, welche befähigt ist, dem Subjekt eines von zwei alternativ auswählbaren Auswahlelementen als einen visuelle Information enthaltenden Stimulus bereitzustellen,
eine Bedienungseinheit, die befähigt ist, in Übereinstimmung mit der Bedienung von dem Subjekt, einen Befehl auszugeben, der anzeigt, daß eines der zwei Auswahlelemente ausgewählt ist, und
eine Steuereinheit, die befähigt ist, die Informationsbereitstellungseinheit zu steuern, um dem Subjekt das Auswahlelement bereitzustellen, und die befähigt ist, Information für den Nachweis des Einsetzens von Demenz auf Basis des eingegebenen Befehls von der Bedienungseinheit zu analysieren, wobei die Steuereinheit aufweist:

eine Stimulusbereitstellungseinheit, die befähigt ist, einen Zielstimulus bereitzustellen, der eine Vielzahl an Malen eines der zwei Auswahlelemente als visuelle Information durch die Informationsbereitstellungseinheit bereitstellt und die Stimulusbereitstellungseinheit befähigt ist, einen Hinweisstimulus bereitzustellen, welcher visuelle Information ist, die eines der zwei Auswahlelemente für eine vorbestimmte Dauer durch die Informationsbereitstellungseinheit anweist, bevor der Zielstimulus für jede der Vielzahl an Malen bereitgestellt wird,
eine Meßeinheit, die befähigt ist, eine Antwortzeit von der Bereitstellung des Zielstimulus durch die Stimulusbereitstellungseinheit bis zum Erhalt des von der Bedienungseinheit ausgegebenen Befehls zu messen,
eine Durchschnittszeitberechnungseinheit, die befähigt ist, einen Durchschnittswert der Antwortzeiten, die von der Meßeinheit gemessen werden, zu berechnen,
eine Speichereinheit, die befähigt ist, vorab Referenzdurchschnittswerte zu speichern, die vorab im Hinblick auf eine Vielzahl von Subjekten, enthaltend einen Demenzpatienten und eine von dem Demenzpatienten verschiedene Person, erstellt wurden, wobei die Werte Durchschnittswerte der Antwortzeiten auf den von der Stimulusbereitstellungseinheit bereitgestellten Stimulus sind und
eine Datenerstellungseinheit, die befähigt ist, Daten zu erstellen, in denen der Durchschnittswert der Antwortzeiten, die im Hinblick auf ein spezifiziertes Subjekt durch die Durchschnittszeitberechnungseinheit erhalten wurden, mit den Referenzdurchschnittswerteri verglichen werden.

2. Vorrichtung für den Nachweis des Einsetzens von Demenz nach Anspruch 1, wobei die Hinweisstimuli einen Gültigkeitshinweisstimulus enthalten, der befähigt ist, vorab das von dem Zielstimulus angezeigte Element anzuweisen, und einen Ungültigkeitshinweisstimulus, der befähigt ist, vorab ein Element anzuweisen, daß gegenteilig zu dem von dem Zielstimulus anzeigten Element ist, die Durchschnittszeitberechnungseinheit befähigt ist, einen Durchschnittswert der Antwortzeiten unter Ausschluß der Antwortzeit auf den Zielstimulus nach Bereitstellung des Ungültigkeitshinweisstimulus zu berechnen und die Speichereinheit befähigt ist, vorab einen Referenzdurchschnittswert unter Ausschluß der Antwortzeit auf den Zielstimulus nach Bereitstellung des Ungültigkeitsstimulus zu speichern.

## Revendications

1. Dispositif de vérification de l'apparition de la démence chez un sujet, le dispositif comprenant :

une unité de fourniture d'informations comprenant une unité de fourniture d'information visuelle pouvant fournir au sujet un parmi deux éléments de choix pouvant être sélectionnés de manière alternée en tant qu'information visuelle comportant un stimulus ;
une unité d'activation capable de délivrer, en fonction d'une activation à partir du sujet, une instruction indiquant que l'un parmi les deux éléments de choix est sélectionné ; et
une unité de commande pouvant commander l'unité de fourniture d'informations dans le but de délivrer au sujet l'élément de choix, et pouvant analyser des informations destinées à vérifier l'apparition de la démence sur la base de l'instruction entrée à partir de l'unité d'activation, dans lequel l'unité de commande comporte :

une unité de fourniture de stimulus pouvant délivrer un stimulus cible délivrant une pluralité de fois l'un des deux éléments de choix en tant qu'information visuelle par l'intermédiaire de l'unité de fourniture d'informations, et l'unité de fourniture de stimulus pouvant délivrer un stimulus signal qui constitue des informations visuelles imposant l'un des deux éléments de choix pendant une période prédéfinie par l'intermédiaire de l'unité de fourniture d'informations, avant de délivrer les stimuli cibles à chacune de la pluralité de fois ;
une unité de mesure pouvant mesurer un temps de réponse à partir de la fourniture du stimulus cible par l'unité de fourniture de stimulus jusqu'à la réception de l'instruction délivrée par l'unité d'activation ;
une unité de calcul de temps moyen pouvant calculer une valeur moyenne des temps de réponse mesurés par l'unité de mesure ;
une unité de mémorisation capable de mémoriser préalablement des valeurs moyennes de référence préparées préalablement par rapport à une pluralité de sujets comportant un patient atteint de démence et une personne autre que le patient atteint de démence, les valeurs étant des valeurs moyennes des temps de réponse au stimulus délivré par l'unité de fourniture de stimulus ; et
une unité de création de données pouvant créer des données, dans laquelle la valeur moyenne des temps de réponse obtenus en ce qui concerne un sujet spécifié par l'unité de calcul de temps moyen est comparée aux valeurs moyennes de référence.

2. Dispositif de vérification de l'apparition de la démence selon la revendication 1, dans lequel :

les stimuli signaux comportent un stimulus signal de validité pouvant imposer préalablement un élément indiqué par le stimulus cible, et un stimulus signal d'invalidité pouvant imposer préalablement un élément opposé à l'élément indiqué par le stimulus cible,
l'unité de calcul de temps moyen peut calculer une valeur moyenne des temps de réponse excluant le temps de réponse au stimulus cible après la fourniture du stimulus signal d'invalidité, et
l'unité de mémorisation peut mémoriser préalablement une valeur moyenne de référence excluant le temps de réponse au stimulus cible après la fourniture du stimulus signal d'invalidité.

# FIG.1

**FIG.2**

CONTROL UNIT ~5

INFORMATION COLLECTION UNIT ~6

STORAGE UNIT ~8

TEST CONDITION SELECTION UNIT ~11

STIMULUS PROVISION UNIT ~12

MEASUREMENT UNIT ~13

SCREEN CONTROL UNIT ~9

DISPLAY ~2

OPERATION UNIT ~4

SOUND OUTPUT UNIT ~10

EAR PAD UNIT ~3

INFORMATION ANALYSIS UNIT

AVERAGE TIME COMPUTATION UNIT ~14

CORRECT ANSWER PERCENTAGE COMPUTATION UNIT ~15

INDEX COMPUTATION UNIT ~16 ~7

DATA CREATION UNIT ~17

EP 2 649 945 B1

FIG.3A      FIG.3B      FIG.3C

## FIG.4

## FIG.5

## FIG.6

FIG.7

# FIG.8

# FIG.9

| CONDITION | STIMULUS | SIGNIFICANT DIFFERENCE | | | | | |
|---|---|---|---|---|---|---|---|
| | | Y-E | Y-M | Y-A | E-M | E-A | M-A |
| AUDIO ATTENTION | AUDIO | *** | *** | *** | *** | *** | *** |
| | AUDIO-VISUAL | *** | *** | *** | *** | *** | *** |
| VISUAL ATTENTION | VISUAL | *** | *** | *** | *** | *** | *** |
| | AUDIO-VISUAL | *** | *** | *** | ** | *** | *** |
| AUDIO -VISUAL ATTENTION | AUDIO | *** | *** | *** | | *** | *** |
| | VISUAL | *** | *** | *** | | *** | *** |
| | AUDIO-VISUAL | *** | *** | *** | | *** | *** |

## FIG.10

## FIG.11

| CONDITION | STIMULUS | SIGNIFICANT DIFFERENCE | | | | | |
|---|---|---|---|---|---|---|---|
| | | Y-E | Y-M | Y-A | E-M | E-A | M-A |
| AUDIO ATTENTION | AUDIO | | | *** | | *** | *** |
| | AUDIO-VISUAL | | | * | | | |
| VISUAL ATTENTION | VISUAL | | * | *** | | * | |
| | AUDIO-VISUAL | | | | | | |
| AUDIO -VISUAL ATTENTION | AUDIO | | | *** | | *** | *** |
| | VISUAL | *** | *** | *** | * | | |
| | AUDIO-VISUAL | | | *** | | *** | *** |

# FIG.12

FIG.13

$P(S_1)+P(S_2)-P(S_1) \times P(S_2)=P(S_1S_2)$

# FIG.14

EP 2 649 945 B1

# FIG.15

FIG.16

EP 2 649 945 B1

# FIG.17

FIG.18

# FIG.19

```
            ┌─────────────┐
            │    START    │
            └──────┬──────┘
                   │   ┌──────────────────────────┐
                   ▼   │                          │
                  ╱─────────────────╲  S1         │
                 ╱  SUBJECT INFORMATION ╲   NO     │
                ╲      INPUT?         ╱────────────┘
                 ╲─────────────────╱
                   │ YES
                   ▼        S2
            ┌──────────────────────────┐
            │ STORE SUBJECT INFORMATION│
            └──────────────┬───────────┘
                   │   ┌──────────────────────────┐
                   ▼   │                          │
                  ╱─────────────────╲  S3         │
                 ╱    START SWITCH     ╲   NO      │
                 ╲    TURNED ON?      ╱────────────┘
                  ╲─────────────────╱
                   │ YES
                   ▼        T
            ┌──────────────────────────┐
            │ INFORMATION COLLECTING PROCESSING │
            └──────────────┬───────────┘
                   │        U
                   ▼
            ┌──────────────────────────┐
            │ INFORMATION ANALYSIS PROCESSING │
            └──────────────┬───────────┘
                   │        S4
                   ▼
            ┌──────────────────────────┐
            │ OUTPUT ANALIZED INFORMATION │
            └──────────────┬───────────┘
                   │
                   ▼
            ┌─────────────┐
            │     END     │
            └─────────────┘
```

# FIG.20

INFORMATION COLLECTING PROCESSING — T

DETERMINE AND STORE
SEQUENCE OF TEST CONDITIONS — T1

RETRIEVE NEXT TEST CONDITION
AND INCREASE TEST COUNTER BY 1 — T2

AUDIO
SELECTIVE ATTENTION
CONDITION? — T3 — NO

YES

AUDIO SELECTIVE ATTENTION
CONDITION TEST — V

VISUAL
SELECTIVE ATTENTION
CONDITION? — T4 — NO

YES

VISUAL SELECTIVE ATTENTION
CONDITION TEST — W

AUDIO-VISUAL
DIVISION ATTENTION
CONDITION? — T5 — NO

YES

AUDIO-VISUAL DIVISION ATTENTION
CONDITION TEST — X

NO — COUNTER IS 3? — T6

YES

RESET TEST COUNTER TO 0 — T7

RETURN

# FIG.21

```
                                              ╭───V
        ┌─────────────────────────────────┐
        │ AUDIO SELECTIVE ATTENTION       │
        │ CONDITION TEST                  │
        └─────────────────────────────────┘
                        │
                        ▼                    ╭───V1
        ┌─────────────────────────────────┐
        │ DISPLAY EXPLANATION SCREEN      │
        └─────────────────────────────────┘
                        │
                        ▼         ╭───V2
                 ╱──────────────╲          NO
                ╱  INPUT OF      ╲──────────┐
                ╲  UNDERSTANDING ╱
                 ╲  MADE?       ╱
                  ╲───────────╱
                        │ YES
                        ▼                    ╭───V3
        ┌─────────────────────────────────┐
        │ INCREASE SESSION COUNTER BY 1   │
        └─────────────────────────────────┘
                        │
                        ▼                    ╭───V4
        ┌─────────────────────────────────┐
        │ DISPLAY CONDITION IMAGE FOR     │
        │ AUDIO SELECTION                 │
        └─────────────────────────────────┘
                        │                    ╭───Y
        ┌─────────────────────────────────┐
        │ STIMULUS PROVISION              │
        │ PROCESSING                      │
        └─────────────────────────────────┘
                        │
                        ▼         ╭───V5
         NO      ╱──────────────╲       YES
      ┌─────────╱  SESSION       ╲──────────┐
      │         ╲  COUNTER IS 3  ╱          │
      │          ╲  ?           ╱           │
      │           ╲───────────╱             │
      │                                     │
      │                                     ▼              ╭───V8
      │                          ┌─────────────────────────────┐
      │         ╭───V6           │ RESET SESSION COUNTER       │
      ▼                          │ AND FREQUENCY               │
  ┌──────────────────────────┐   │ COUNTER TO 0                │
  │ HIDE CONDITION IMAGE     │   └─────────────────────────────┘
  │ FOR AUDIO SELECTION      │                │
  │ AND DISPLAY INFORMATION  │                ▼              ╭───V9
  │ ON RESTART AFTER         │   ┌─────────────────────────────┐
  │ PRESET PERIOD            │   │ HIDE CONDITION IMAGE        │
  └──────────────────────────┘   │ FOR AUDIO SELECTION         │
            │                     └─────────────────────────────┘
            ▼      ╭───V7                      │
        ╱──────────────╲    NO                 ▼
       ╱  PRESET        ╲──────┐         ╭───────────╮
       ╲  PERIOD ELAPSED╱      │         │  RETURN   │
        ╲  ?           ╱       │         ╰───────────╯
         ╲───────────╱
              │ YES
```

# FIG.22

```
                                              ┌─W
                        ┌─────────────────────────────┐
                        │ VISUAL SELECTIVE ATTENTION │
                        │      CONDITION TEST         │
                        └─────────────────────────────┘
                                      │
                                      ▼           ┌─W1
                        ┌─────────────────────────────┐
                        │  DISPLAY EXPLANATION SCREEN │
                        └─────────────────────────────┘
                                      │
                                      ▼
                              ┌──────────────┐
                              │   INPUT OF   │   ┌─V2
                              │ UNDERSTANDING│      NO
                              │    MADE?     │
                              └──────────────┘
                                   │ YES
                                   ▼              ┌─V3
                        ┌─────────────────────────────┐
                        │ INCREASE SESSION COUNTER BY 1│
                        └─────────────────────────────┘
                                   │
                                   ▼              ┌─W4
                        ┌─────────────────────────────┐
                        │ DISPLAY CONDITION IMAGE FOR │
                        │      VISUAL SELECTION        │
                        └─────────────────────────────┘
                                   │
                                   ▼              ┌─Y
                        ┌─────────────────────────────┐
                        │   STIMULUS PROVISION        │
                        │      PROCESSING             │
                        └─────────────────────────────┘
                                   │
                                   ▼          ┌─V5
                       NO  ┌──────────────┐  YES
                           │   SESSION    │
                           │ COUNTER IS 3 │
                           │      ?       │
                           └──────────────┘
```

RESET SESSION COUNTER AND FREQUENCY COUNTER TO 0    ─V8

HIDE CONDITION IMAGE FOR VISUAL SELECTION AND DISPLAY INFORMATION ON RESTART AFTER PRESET PERIOD    ─W6

PRESET PERIOD ELAPSED ?    ─V7    NO    YES

HIDE CONDITION IMAGE FOR VISUAL SELECTION    ─W9

RETURN

# FIG.23

```
                                                              ┌─X
                           ┌──────────────────────────────┐
                           │      AUDIO-VISUAL DIVISION     │
                           │   ATTENTION CONDITION TEST     │
                           └──────────────┬─────────────────┘
                                          │              ┌─X1
                           ┌──────────────▼─────────────────┐
                           │   DISPLAY EXPLANATION SCREEN    │
                           └──────────────┬─────────────────┘
                                          │
                                          ▼         ┌─V2
                              ◇──────────────────────◇  NO
                                 INPUT OF
                                 UNDERSTANDING
                                 MADE?
                                          │ YES
                                          │                  ┌─V3
                           ┌──────────────▼─────────────────┐
                           │  INCREASE SESSION COUNTER BY 1  │
                           └──────────────┬─────────────────┘
                                          │              ┌─X4
                           ┌──────────────▼─────────────────┐
                           │    DISPLAY CONDITION IMAGE      │
                           │      FOR AUDIO-VISUAL           │
                           └──────────────┬─────────────────┘
                                          │              ┌─Y
                           ┌──────────────▼─────────────────┐
                           │    STIMULUS PROVISION           │
                           │       PROCESSING                │
                           └──────────────┬─────────────────┘
                                          ▼        ┌─V5
                     NO      ◇──────────────────────◇  YES
                                 SESSION
                                 COUNTER IS 3
                                 ?
```

- AUDIO-VISUAL DIVISION ATTENTION CONDITION TEST — X
- DISPLAY EXPLANATION SCREEN — X1
- INPUT OF UNDERSTANDING MADE? — V2 — NO
- YES
- INCREASE SESSION COUNTER BY 1 — V3
- DISPLAY CONDITION IMAGE FOR AUDIO-VISUAL — X4
- STIMULUS PROVISION PROCESSING — Y
- SESSION COUNTER IS 3 ? — V5 — NO / YES
- HIDE CONDITION IMAGE FOR AUDIO-VISUAL AND DISPLAY INFORMATION ON RESTART AFTER PRESET PERIOD — X6
- PRESET PERIOD ELAPSED ? — V7 — NO / YES
- RESET SESSION COUNTER AND FREQUENCY COUNTER TO 0 — V8
- HIDE CONDITION IMAGE FOR AUDIO-VISUAL — X9
- RETURN

FIG.24

STIMULUS PROVISION PROCESSING — Y

↓

DECIDE STIMULUS OUTPUT PATTERN — Y1

↓

RETRIEVE NEXT STIMULUS — Y2

↓

Y3 — AUDIO STIMULUS ? — NO

YES ↓

Y4 — OUTPUT AUDIO STIMULUS FOR PRESET PERIOD AND STOP

↓

Y5 — VISUAL STIMULUS ? — NO

YES ↓

Y6 — OUTPUT VISUAL STIMULUS FOR PRESET PERIOD AND STOP

↓

Y7 — AUDIO-VISUAL STIMULUS? — NO

YES ↓

Y8 — OUTPUT AUDIO-VISUAL STIMULUS FOR PRESET PERIOD AND STOP

↓

Y9 — RESPONSE INPUT MADE? — NO

YES ↓

Y11 — PRESET PERIOD ELAPSED FROM STIMULUS STOP ? — NO

Y10 — STORE RESPONSE TIME AND RESPONSE CONTENTS

YES ↓

Y12 — STORE INFORMATION ON INVALIDITY OF RESPONSE

↓

Y13 — INCREASE FREQUENCY COUNTER BY 1

↓

Y14 — FREQUENCY COUNTER IS 72? — NO

YES ↓

RETURN

# FIG.25

```
                                              ┌─U
┌────────────────────────────────────────────────┐
│         INFORMATION ANALYSIS PROCESSING          │
└────────────────────────────────────────────────┘
                        │
                        ▼                      ┌─U1
┌────────────────────────────────────────────────┐
│        DETERMINE WHETHER RESPONSE IS CORRECT     │
└────────────────────────────────────────────────┘
                        │
                        ▼                      ┌─U2
┌────────────────────────────────────────────────┐
│          COMPUTE AND STORE AVERAGE VALUE OF      │
│                  RESPONSE TIMES                  │
└────────────────────────────────────────────────┘
                        │
                        ▼                      ┌─U3
┌────────────────────────────────────────────────┐
│    CREATE AND STORE COMPARISON DATA, IN WHICH    │
│  COMPUTED AVERAGE VALUE OF RESPONSE TIMES IS     │
│        COMPARED WITH PREVIOUSLY STORED           │
│          AVERAGE VALUES OF RESPONSE TIMES        │
└────────────────────────────────────────────────┘
                        │
                        ▼                      ┌─U4
┌────────────────────────────────────────────────┐
│          COMPUTE AND STORE PERCENTAGE OF         │
│                 CORRECT ANSWERS                  │
└────────────────────────────────────────────────┘
                        │
                        ▼                      ┌─U5
┌────────────────────────────────────────────────┐
│           CREATE AND STORE COMPARISON DATA,      │
│    IN WHICH COMPUTED PERCENTAGE OF CORRECT       │
│  ANSWERS IS COMPARED WITH PREVIOUSLY STORED      │
│       PERCENTAGES OF CORRECT ANSWERS             │
└────────────────────────────────────────────────┘
                        │
                        ▼                      ┌─U6
┌────────────────────────────────────────────────┐
│ COMPUTE AND STORE CUMULATIVE DISTRIBUTION        │
│        PROBABILITY OF RESPONSE TIMES             │
└────────────────────────────────────────────────┘
                        │
                        ▼                      ┌─U7
┌────────────────────────────────────────────────┐
│         COMPUTE AND STORE ESTIMATE VALUE         │
└────────────────────────────────────────────────┘
                        │
                        ▼                      ┌─U8
┌────────────────────────────────────────────────┐
│  COMPUTE AND STORE DIFFERENCE BETWEEN            │
│   CUMULATIVE DISTRIBUTION PROBABILITIES          │
│            AND ESTIMATE VALUES                   │
└────────────────────────────────────────────────┘
                        │
                        ▼                      ┌─U9
┌────────────────────────────────────────────────┐
│      CREATE AND STORE COMPARISON DATA,           │
│ IN WHICH COMPUTED SUBTRACTION VALUE IS           │
│     COMPARED WITH PREVIOUSLY STORED              │
│            SUBTRACTION VALUES                    │
└────────────────────────────────────────────────┘
                        │
                        ▼
                  (  RETURN  )
```

# FIG.26

# FIG.27

CONTROL UNIT ~25

STORAGE UNIT ~8

INFORMATION COLLECTION UNIT ~26

STIMULUS PROVISION UNIT ~12

MEASUREMENT UNIT ~13

OPERATION UNIT ~24

SCREEN CONTROL UNIT ~9

DISPLAY ~2

INFORMATION ANALYSIS UNIT

AVERAGE TIME COMPUTATION UNIT ~14

CORRECT ANSWER PERCENTAGE COMPUTATION UNIT ~15

DATA CREATION UNIT ~17 ~27

EP 2 649 945 B1

# FIG.28

BACKGROUND
(1000ms)

CUE STIMULUS
(200ms)

INTERVAL
(200,400,800ms)

TARGET STIMULUS
(100ms)

BACKGROUND
(1500,1300,900ms)

TASK
(3000ms)

EP 2 649 945 B1

FIG.29

FIG.30

Ta2

10%

Ta1

90%

# FIG.31

# FIG.32

|  | EC<br>(HEALTHY ELDERLY PEOPLE) | AD<br>(DEMENTIA PATIENTS) |
|---|---|---|
| NUMBER OF SUBJECTS | 16 | 11 |
| MALE/FEMALE | 10/16 | 7/4 |
| AGE | 72.5±3.9 | 73.4±6.7 |
| CDR SCORE | 0±0 | 1.4±0.4 |
| MMSE SCORE | 29.5±0.5 | 22.1±2.4 |

# FIG.33

```
┌─────────────────────────────┐
│ INFORMATION COLLECTING      │──T
│        PROCESSING           │
└─────────────────────────────┘
              │
              ▼           T11
┌─────────────────────────────┐
│   DECIDE TASK COMBINATION   │
└─────────────────────────────┘
              │
              ▼           T12
┌─────────────────────────────┐
│   DISPLAY BACKGROUND IMAGE  │
└─────────────────────────────┘
              │          ◄──────── ①
              ▼
┌─────────────────────────────┐
│ INCREASE TASK COUNTER BY 1  │──T13
└─────────────────────────────┘
              │
              ▼           T14
         ╱STANDBY TIME╲
NO ◄────╱   ARRIVED?    ╲
         ╲             ╱
              │ YES
              ▼           T15
┌─────────────────────────────┐
│      DISPLAY CUE IMAGE      │
└─────────────────────────────┘
              │
              ▼           T16
         ╱  CUE DISPLAY  ╲
NO ◄────╱ TIME LIMIT ARRIVED
         ╲      ?       ╱
              │ YES
              ▼           T17
┌─────────────────────────────┐
│       HIDE CUE IMAGE        │
└─────────────────────────────┘
              │
              ▼           T18
         ╱   INTERVAL    ╲
NO ◄────╱ TIME LIMIT ARRIVED
         ╲      ?       ╱
              │ YES      Z
┌─────────────────────────────┐
│   RESPONSE RECEPTION        │
│        PROCESSING           │
└─────────────────────────────┘
              │
              ▼           T19
         ╱    STANDBY    ╲
NO ◄────╱ TIME LIMIT ARRIVED
         ╲      ?       ╱
              │ YES
```

```
        T20
    ╱   INTERVAL    ╲
   ╱ BETWEEN TASKS   ╲── NO
    ╲    EXIST?     ╱
         │ YES
        T21
    ╱   INTERVAL    ╲
   ╱ TIME LIMIT ARRIVED ── NO
    ╲      ?       ╱
         │ YES
        T22
    ╱     TASK      ╲
   ╱  COUNTER IS     ╲── NO ──► ①
    ╲     90?       ╱
         │ YES
    ┌──────────┐
    │  RETURN  │
    └──────────┘
```

# FIG.34

RESPONSE RECEPTION
PROCESSING — Z

DISPLAY TARGET IMAGE — Z1

② →

RESPONSE INPUT
MADE? — Z2

NO →

YES

DISPLAY
TIME LIMIT ARRIVED
? — Z5

NO →

YES

HIDE TARGET IMAGE — Z6

RESPONSE
TIME LIMIT ARRIVED
? — Z7

NO → ②

YES

STORE INFORMATION OF
RESPONSE INVALIDITY — Z8

STORE RESPONSE TIME
AND RESPONSE CONTENTS — Z3

HIDE TARGET IMAGE — Z4

RETURN

# FIG.35

```
                                                    ┌─ U
┌─────────────────────────────────────────────┐
│ INFORMATION ANALYSIS PROCESSING │            │
└─────────────────────────────────────────────┘
                        │
                        ▼                         ┌─ U11
┌─────────────────────────────────────────────┐
│   DETERMINE WHETHER RESPONSES ARE           │
│       CORRECT AND STORE RESULTS             │
└─────────────────────────────────────────────┘
                        │
                        ▼                         ┌─ U12
┌─────────────────────────────────────────────┐
│           COMPUTE AND STORE                 │
│     PERCENTAGE OF CORRECT ANSWERS           │
└─────────────────────────────────────────────┘
                        │
                        ▼                         ┌─ U13
┌─────────────────────────────────────────────┐
│           COMPUTE AND STORE                 │
│     AVERAGE VALUE OF RESPONSE TIMES         │
└─────────────────────────────────────────────┘
                        │
                        ▼                         ┌─ U14
┌─────────────────────────────────────────────┐
│ CREATE AND STORE COMPARISON DATA,           │
│  IN WHICH COMPUTED AVERAGE VALUE            │
│ OF RESPONSE TIMES IS COMPARED WITH          │
│ PREVIOUSLY STORED AVERAGE VALUES            │
│           OF RESPONSE TIMES                 │
└─────────────────────────────────────────────┘
                        │
                        ▼
                  (  RETURN  )
```

FIG.36

# FIG.37

EP 2 649 945 B1

# FIG.38

# FIG.39

EP 2 649 945 B1

# FIG.40

FIG.41

# FIG.42

# FIG.43

INCREASE TASK COUNTER BY 1 — T13

START DETECTION BY fMRI — T131

STANDBY TIME ARRAIVED? — T14
NO
YES

STANDBY TIME LIMIT ARRAIVED ? — T19
NO
YES

END DETECTION BY fMRI — T191

STORE DETECTION RESULT BY fMRI — T192

INTERVAL BETWEEN TASKS EXIST? — T20
NO
YES

START DETECTION BY fMRI — T201

T21 — INTERVAL TIME LIMIT ARRIVED ?
NO
YES

END DETECTION BY fMRI — T211

STORE DETECTION RESULT BY fMRI — T212

TO STEP T22

# FIG.44

U

INFORMATION ANALYSIS PROCESSING

↓

U11

DETERMINE WHETHER RESPONSES
ARE CORRECT AND STORE RESULTS

↓

U12

COMPUTE AND STORE PERCENTAGE
OF CORRECT ANSWERS

↓

U13

COMPUTE AND STORE AGERAGE VALUE
OF RESPONSE TIMES

↓

U14

CREATE AND STORE COMPARISON DATA,
IN WHICH COMPUTED AGERAGE VALUE OF
RESPONSE TIMES IS COMPARED WITH
PREVIOUSLY STORED AVERAGE VALUE OF
RESPONSE TIMES

↓

U15

COMPUTE BOLD SIGNAL CHANGE RATE
ON BASIS OF fMRI DETECTION VALUE

↓

U16

CREATE AND STORE COMPARISON DATA,
IN WHICH COMPUTED BOLD SIGNAL CHANGE
RATE IS COMPARED WITH PREVIOUSLY STORED
REFERENCE ANALYSIS VALUE

↓

RETURN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006087743 A

### Non-patent literature cited in the description

- Modality-specific selective attention attenuates multisensory integration. **J L MOZOLIC et al.** EXPERIMENTAL BRAIN RESEARCH. SPRINGER, BERLIN, DE, 08 August 2007, vol. 184, 39-52
- **JENNIFER L. MOZOLIC ; CHRISTINA E. HUGENSCHMIDT ; ANN M. PEIFFER ; PAUL J. LAURIENTI.** Modality-specific selective attention attenuates audiovisual integration. *Experimental Brain Research,* 2008, vol. 184, 39-52
- **PAUL J. LAURIENTI ; JONATHAN H. BURDETTE ; JOSEPH A. MALDJIAN ; MARK T. WALLACE.** Enhanced audiovisual integration in older adults. *Neurobiology of Aging,* 2006, vol. 27, 1155-1163
- **PIETRO PIETRINI ; GENE E. ALEXANDER ; MAURA L. FUREY ; ALESSIO DARN ; MARC J. MENTIS ; BARRY HORWITZ ; MARIO GUAZZEHHI ; MARK B. SCHAPIRO ; STANLEY I. RAPOPORT.** Cerebral Metabolic Response to Passive Audiovisual Stimulation in Patients with Alzheimer's Disease and Healthy Volunteers Assessed by PET. *THE JOURNAL OF NUCLEAR MEDICINE,* 2000, vol. 41, 575-583
- **J.J. DE JONG ; P.P.G. HODIAMONT ; J. VAN DEN STOCK ; B. DE GELDER.** Audiovisual emotion recognition in schizophrenia: Reduced integration of facial and vocal affect. *SCHIZOPHRENIA RESEARCH,* 2009, vol. 107, 286-293
- **JEFF MILLER.** *COGNITIVE PSYCHOLOGY,* 1982, vol. 14, 247-279